# EUROPEAN PATENT APPLICATION

(11) **EP 1 386 894 A1**
(43) Date of publication of application: **04.02.2004**
(21) Application number: 01941025.7
(22) Date of filing: 13.06.2001
(51) Int. Cl.: C04B 12/02, C04B 28/34, A61L 27/12, A61K 6/06, B28C 5/18

(54) **KNEADED PRODUCT CONTAINING CALCIUM PHOSPHATE CEMENT AND METHOD FOR PREPARATION THEREOF**

(30) Priority: 28.03.2001 JP 2001094039; 28.03.2001 JP 2001094040; 28.03.2001 JP 2001094041
(71) Applicant: MITSUBISHI MATERIALS CORPORATION, Chiyoda-ku, Tokyo 100-8117 (JP)
(72) Inventor: HIRANO, Masahiro, c/o Mitsubishi Materials Corp., Yokozemachi,Chichibu-gun,Saitama-ken (JP); TAKEUCHI, Hiroyasu, c/o Mitsubishi Materials Corp., Tokyo100-8117 (JP); ASAOKA, Nobuyuki, c/o Mitsubishi Materials Corp., Yokozemachi , Chichibu-gun ,Saitama-ken (JP)
(74) Representative: Gille Hrabal Struck Neidlein Prop Roos
(86) International application number: PCT/JP2001/004999
(87) International publication number: WO 2002/079112

(57) **Abstract**

According to the process for preparing a kneaded calcium phosphate cement mixture of the present invention, a powder is kneaded with a liquid by simultaneously revolving and rotating a vessel containing a material to be kneaded, which is prepared by mixing a powder composition containing a calcium phosphate powder with a hardening liquid. When a specific surface area of the calcium phosphate powder is from 0.3 to 2.0 m²/g, a weight mixing ratio of the powder to the liquid (P/L) is preferably from 3.0 to 5.0. When the specific surface area is from 2.0 to 4.0 m²/g, P/L is preferably from 1.5 to 3.5.

## Description

### TECHNICAL FIELD

The present invention relates to a kneaded calcium phosphate cement mixture which can be used in medical fields, including oral surgery.

### BACKGROUND ART

In Japanese Patent Application, First Publication No. Sho 64-37445 and Japanese Patent Application, First Publication No. Hei 4-328185, calcium phosphate cements, which can be utilized as a biomedical cement, have hitherto been known. These calcium phosphate cements can be used in filling defective portions of bone and fixation and restoration of bone fractured portions because they are hardened at an approximately neutral pH and do not stimulate the living body. To stably use calcium phosphate cements for the living body, however, a high strength is sometimes required depending on the position of application. These calcium phosphate cements are usually hardened at 37°C within about 10 minutes, but are required to harden within a shorter time depending on the purposes, and therefore, the use thereof has been limited. Since a powder is usually kneaded with a liquid using a mortar and a pestle, the operation is complicated and they are likely to be contaminated with saprophytic bacteria and impurities.

To enhance the strength of a cement filler material, a mixing ratio P/L of the powder to the liquid (weight mixing ratio of the powder to the liquid, hereinafter referred to as P/L or a powder-liquid ratio) on kneading may be increased. When kneading is conducted in a high powder-liquid ratio, the fluidity of the kneaded mixture is lowered, thus making it difficult to obtain a uniform kneaded mixture. Therefore, there has been made a trial of increasing the powder-liquid ratio on kneading by enhancing the fluidity using spherical powder particles (for example, Japanese Patent Application, First Publication No. Hei 1-176252 and Japanese Patent Application, First Publication No. Hei 5-319891).

However, the process of introducing powders into an impact apparatus in a high speed air flow (manufactured by Nara Machinery Co., Ltd., under the trade name "NARA HYBRIDIZER") and applying mechanical impact to the powders in an impact chamber, or the process of introducing powders into a plasma arc generated by high frequency plasma in an argon-hydrogen mixed gas atmosphere, melting a portion of the surface of the powders, and solidifying a portion of the powders scattering in the plasma arc with cooling, thereby spheroidizing the powders, must be used to obtain spherical powders and these processes are not practical because they are extremely complicated and the cost is high.

### DISCLOSURE OF THE INVENTION

An object of the present invention is to provide a kneaded calcium phosphate cement mixture having a high viscosity, which is useful as a biomedical prosthetic appliance, because it requires a short hardening time and achieves a markedly high strength after filling. Another object of the present invention is to provide a process for preparing a kneaded calcium phosphate cement mixture having a high viscosity, which is capable of kneading a powder with a liquid at a high powder-liquid ratio in a simple manner at a low cost, and is less likely to cause contamination with saprophytic bacteria and impurities. The kneaded calcium phosphate cement mixture of the present invention can be used as a dental cement, a gypsum substitute such as dental gypsum, and a building cement, in addition to a biomedical prosthetic appliance.

To achieve the objects described above, according to the process for preparing a kneaded calcium phosphate cement mixture of the present invention, a powder is kneaded with a liquid by simultaneously revolving and rotating a vessel containing a material to be kneaded, which is prepared by mixing a powder composition containing a calcium phosphate powder with a hardening liquid.

The powder may be kneaded with the liquid by simultaneously revolving and rotating the vessel in the state of being inclined by 20 to 50 degrees. The powder may be kneaded with the liquid by revolving the vessel at 1500 rpm or more while rotating it at 300 rpm in the state of being inclined by 20 to 50 degrees.

The powder composition may be composed only of a calcium phosphate powder. The calcium phosphate powder may be a powder of α-type tricalcium phosphate or tetracalcium phosphate, or a powder composition which contains at least one of α-type tricalcium phosphate and tetracalcium phosphate as an essential component and also contains dicalcium phosphate and monocalcium phosphate as an arbitrary component.

The powder composition may contain a calcium phosphate powder and a magnesium-containing compound. As the magnesium-containing compound, at least one of magnesium phosphate and magnesium sulfate can be used.

The powder composition may contain a calcium phosphate powder and a pyrophosphate. The pyrophosphate may be at least one of sodium pyrophosphate and potassium pyrophosphate. In this case, the powder composition may further contain at least one of magnesium phosphate and magnesium sulfate.

The hardening liquid may be an aqueous solution containing at least one selected from succinate, chondroitin sulfate, and hydrogensulfite.

The vessel may be a double vessel comprising an outer vessel and an inner vessel housed in the outer vessel, and furthermore, the outer vessel and the inner vessel may be provided with a locking mechanism for preventing a relative position of the inner vessel housed in the outer vessel from changing.

The inner vessel may have such a constitution that it comprises a vessel body having a first opening for discharging a kneaded mixture and a second opening into which an extruding member for extruding the kneaded mixture in the inner vessel through the first opening, and a cover member which detachably seals the first opening and the second opening of the vessel body.

When using the double vessel described above, kneading is preferably conducted in the state where the outer vessel, the inner vessel and the material to be kneaded, which is contained in the inner vessel, are sterilized.

The kneaded calcium phosphate cement mixture of the present invention may be obtained by mixing a calcium phosphate powder having a specific surface area of 0.3 to 2.0 m²/g with a hardening liquid in a weight mixing ratio of the powder to the liquid (P/L) of 3.0 to 5.0 and treating the mixture using the kneading process described above. Alternatively, it may be obtained by mixing a calcium phosphate powder having a specific surface area of 2.0 to 4.0 m²/g with a hardening liquid in a weight mixing ratio of the powder to the liquid (P/L) of 1.5 to 3.5 and treating the mixture using the kneading process described above.

In the kneaded calcium phosphate cement mixture of the present invention, a consistency is preferably from 18 to 22 mm. The amount of bubbles in the kneaded calcium phosphate cement mixture of the present invention is preferably 10% or less based on the volume of the kneaded mixture.

The hardened biomedical calcium phosphate bone filling cement material of the present invention is obtained by solidifying the kneaded calcium phosphate cement mixture described above and has a compression strength of 60 MPa or more.

### BRIEF DESCRIPTION OF THE DRAWINGS

Fig. 1 is a sectioned front view showing one example of a double vessel for kneading which is suited for use in the process of the present invention.
Fig. 2 is a sectioned front view showing another example of a double vessel for kneading which is suited for use in the process of the present invention.

### BEST MODE FOR CARRYING OUT THE INVENTION

Preferred examples of the present invention will be described below with reference to the accompanying drawings. The present invention is not limited to the following respective examples and, for example, constituent elements of these examples may be appropriately used in combination.

According to the process for preparing a kneaded calcium phosphate cement mixture of the present invention, a powder is kneaded with a liquid by simultaneously revolving and rotating a vessel containing a material to be kneaded, which is prepared by mixing a powder composition containing a calcium phosphate powder with a hardening liquid. Preferred examples will be concretely described below.

### First Example

In the first example of the present invention, the powder composition is substantially composed only of a calcium phosphate powder. The calcium phosphate powder used in the present invention is preferably a powder of α-type tricalcium phosphate or tetracalcium phosphate, or a powder composition which contains at least one of α-type tricalcium phosphate and tetracalcium phosphate as an essential component and also contains dicalcium phosphate and monocalcium phosphate as an arbitrary component. The α-type tricalcium phosphate and the tetracalcium phosphate have a hydraulic property and have a function of hardening a kneaded cement mixture prepared by adding a hardening liquid to a powder composition containing at least one of them and kneading the mixture. The dicalcium phosphate and monocalcium phosphate as the arbitrary component have a function of accelerating hardening of the kneaded cement mixture, enhancing an initial strength, and improving the biocompatibility.

For example, the calcium phosphate powder composition used in the present invention contains 100 to 50% by weight, preferably 95 to 60% by weight, and most preferably 90 to 70% by weight, of at least one of α-type tricalcium phosphate and tetracalcium phosphate as the essential component and also contains 0 to 50% by weight, and preferably 5 to 20% by weight, of dicalcium phosphate and 0 to 50% by weight, and preferably 3 to 15% by weight, of monocalcium phosphate as the arbitrary component. The calcium phosphate powder composition further containing 2 to 10% by weight, and preferably 3 to 7% by weight, of a hydrate of dicalcium phosphate and tricalcium phosphate may be used. The composition (combination), the firing temperature and the average particle diameter of the calcium phosphate powder composition can be appropriately selected before use, taking into account the biocompatibility and purposes. The specific surface area is decided within a certain range depending on the content thereof.

In the present invention, the calcium phosphate powder composition having a specific surface area of 0.3 to 2.0 m²/g, or of 2.0 to 4.0 m²/g, is preferably used.

The specific surface area is an important parameter having a strong relationship with the particle size, the reactivity and the fluidity of the calcium phosphate powder composition. When the specific surface area is less than 0.3 m²/g, the hardening time is prolonged because of poor reactivity, and the resulting product is not suited for use as a biomedical cement. On the other hand, when the specific surface area exceeds 4.0 m²/g, the fluidity of the resulting kneaded paste is lowered because of small particle size and high reactivity, and therefore a weight mixing ratio P/L of the powder to the liquid cannot be increased to a range from 1.5 to 5.0.

As the hardening liquid used in the present invention, there can be used water, or an aqueous solution containing water-soluble inorganic salts and/or organic salts for the purpose of improving the ease of kneading, the stability of a kneaded mixture, pH control and the hardening speed on mixing or kneading with the calcium phosphate powder composition, or an aqueous solution containing other components, for example, pharmaceutically acceptable additives such as preservatives and disinfectants.

Examples of the water-soluble inorganic salts and/or organic salts include succinate such as disodium succinate hexahydrate, chondroitin sulfate such as sodium chondroitin sulfate, and hydrogensulfite such as sodium hydrogensulfite. In the case of incorporating these salts, the amount of disodium succinate hexahydrate is within a range from 0 to 30% by weight, and preferably from 5 to 25% by weight; the amount of sodium chondroitin sulfate is within a range from 0 to 10% by weight, and preferably from 3 to 7% by weight; and the amount of sodium hydrogensulfite is within a range from 0 to 10% by weight, and preferably from 0.2 to 0.5% by weight.

The hardening liquid is preferably prepared in a sterilized condition by filling in a sealable vessel having a proper volume such as a vial and sterilizing using a high pressure steam sterilization or radiation sterilization process, or disinfecting a liquid using a bacterial filter, filling the sterilized liquid in a sterilized vessel, and sealing the vessel.

The powder is usually kneaded with the liquid using a mortar and a pestle. In the case in which the powder having a specific surface area of 2.0 to 4.0 m²/g is kneaded with the liquid manually, a mixing ratio P/L of the powder to the liquid, capable of uniformly kneading is at most about 2.0. Since kneading using the mortar and the pestle is conducted in an open system as a matter of course, contamination with saprophytic bacteria and impurities is likely to occur frequently.

The present inventors have intensively researched and have found that, by filling a sterilized vessel with a powder and a liquid and rotating the vessel while revolving the vessel, the powder can be easily kneaded with the liquid due to only a centrifugal force of the revolution without requiring any manual action. Moreover, a uniform kneaded cement mixture could be obtained in the sterilized condition, even though the time required for kneading is only about 5 to 40 seconds.

It has also be found that the hardening time is markedly reduced by friction heat between the kneaded mixture and the wall surface of the vessel generated as a result of the revolution, in addition to a high powder-liquid ratio, and that the fluidity of the kneaded mixture is markedly improved by the deaeration effect in the kneaded mixture during the revolution.

The vessel can be revolved and rotated using "SPINKLE" (trade name, manufactured by J.MORITA TOKYO MFG. CORP.) and "AWATORI RENTARO" (trade name, manufactured by THINKY Corp.). The objective uniform pasty or clay-like kneaded cement mixture having a high viscosity can be easily obtained by sterilizing a kneading vessel attached to each product with an ethylene oxide gas, filling the sterilized vessel with a calcium phosphate powder composition and a hardening liquid in a powder-liquid ratio of 1.5 to 3.5, or of 3.0 to 5.0, and kneading them for about 5 to 40 seconds.

Kneading due to the rotation and revolution movement as well as deaeration by these apparatuses are described in Japanese Patent Application, First Publication No. Hei 11-309358 and Japanese Patent Application, First Publication No. 2000-271465. Although these publications describe the use in kneading of dental impression materials and powders and the deaeration of bubbles in the slurry under the conditions of a revolution speed of 1000 rpm and a rotation speed within a range from 500 to 1000 rpm, the matter of a decrease in hardening time and an increase in strength due to the kneading and aeration are not reported. These publications do not describe that the subject is a biomedical calcium phosphate bone filling cement and that kneading is conducted in the sterilized condition using a double vessel and kneading is conducted at a high powder-liquid ratio.

In view of provision of a kneaded cement mixture in the sterilized condition, the kneading vessel is preferably a double vessel comprising an outer vessel and an inner vessel housed in the outer vessel. First, the sterilized inner vessel is filled with a powder and a liquid (previously sterilized). A lid is put on the inner vessel to prevent the interior from being contaminated, and then the whole inner vessel is set in a sterilized outer vessel and a lid is put on the outer vessel. The outer vessel is set in the "SPINKLE" or "AWATORI RENTARO" described above and the powder is kneaded with the liquid by simultaneously revolving and rotating. Since the whole apparatus cannot be sterilized, the outer surface of the outer vessel must be in the sterilized condition. However, when the whole external vessel is taken out of the apparatus and the lid of the outer vessel is removed, and then the inner vessel is taken out in the sterilized condition, after the completion of the kneading, both the interior and outer surface surface of the inner vessel are maintained in the sterilized condition. Therefore, in case the operator himself takes out the kneaded mixture included therein on operation, he can easily take out the kneaded mixture while maintaining the sterilized condition. It is necessary to provide either of the outer vessel and the inner vessel with a locking mechanism for preventing revolution of the inner vessel housed in the outer vessel.

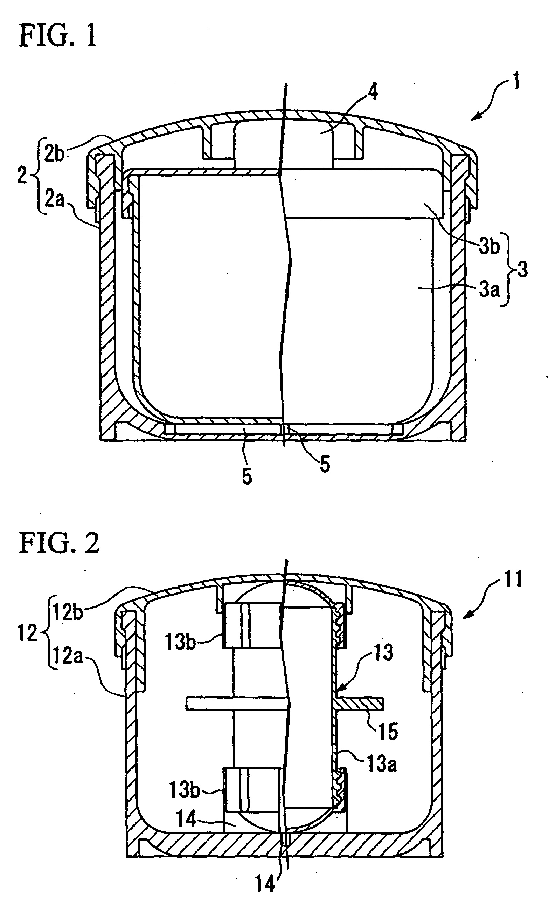

Fig. 1 shows one example of a double vessel suited to carry out the process of the present invention. This double vessel 1 is composed of an outer vessel 2 comprising an outer vessel body 2a and an outer lid 2b which detachably covers a top opening of the outer vessel, and an inner vessel 3 comprising an inner vessel body 3a which can be housed in the outer vessel 2 and a inner lid 3b which detachably covers a top opening of the inner vessel and is equipped with a knob 4. The inner vessel 3 is housed in the outer vessel body 2a in the state where the inner lid 3b is put thereon, and the top end of the knob 4 of the inner vessel 3 comes close to or contacts with the inner surface of the outer lid 2b in the state where the outer lid 2b is put thereon. On the bottom surface of the inner side of the outer vessel body 2a, a cross-shaped recess portion is provided, while a cross-shaped protrusion 5 (locking mechanism) to be fit into the cross-shaped recess portion is provided on the bottom surface of the outer side of the inner vessel body 3a, and the inner vessel 3 can be held in the outer vessel 2 without being revolved by housing the inner vessel 3 in the outer vessel 2 and fitting the protrusion 5 into the recess portion. In this double vessel 1, a sterilized calcium phosphate powder and a sterilized liquid are charged in a predetermined powder-liquid ratio in the sterilized inner vessel body 3a and the inner lid 3b is put thereon. The inner vessel 3 is housed in the sterilized outer vessel 2 and, after fitting the protrusion 5 into the recess portion and putting the outer lid 2b thereon, the outer vessel 2 is mounted in the aforementioned kneading apparatus and the powder is kneaded with the liquid by simultaneously revolving and rotating. After the completion of kneading, the inner vessel 3 is taken out and the inner lid 3b is removed, and then the kneaded cement mixture in the inner vessel is used, thus making it possible to use the kneaded cement mixture in the sterilized condition.

Fig. 2 shows another example of a double vessel suited to carry out the process of the present invention. This double vessel 11 is composed of an outer vessel 12 comprising an outer vessel body 12a and an outer lid 12b which detachably covers a top opening of the outer vessel, and an inner vessel 13 comprising a generally cylindrical inner vessel body 13a having a first opening for discharging a kneaded mixture and a second opening into which an extruding member for extruding the kneaded mixture in the inner vessel through the first opening is inserted, and two cover members 13b which detachably seal the first and second openings of the inner vessel body 13a.

In the center portion of the bottom surface of the inner side of the outer vessel body 12a, a cross-shaped recess portion is provided, while the cover member 13b at the lower portion of the inner vessel 13 is provided with a cross-shaped protrusion 14 (locking mechanism) to be fit into the cross-shaped recess portion, and the inner vessel 13 can be held in the outer vessel 12 without being revolved by housing the inner vessel 13 in the outer vessel 12 and fitting the protrusion 14 into the recess portion. A flange 15, which serves as a holder in the case of using the inner vessel 13 as an injector, is formed at the center of the inner vessel body 13a.

This double vessel 11 is sterilized and, after opening the upper cover member 13b, a sterilized calcium phosphate powder and a sterilized liquid are charged in a predetermined powder-liquid ratio in the sterilized inner vessel body 13a and the upper cover member 13b is closed. Similarly, the inner vessel 13 is housed in the sterilized outer vessel 12 and, after fitting the protrusion 14 of the lower cover member 13b into the recess portion formed in the outer vessel body 12a and putting the outer lid 12b thereon, the outer vessel 12 is mounted in the aforementioned kneading apparatus and the powder is kneaded with the liquid by simultaneously revolving and rotating. After the completion of kneading, the inner vessel 13 is taken out from the outer vessel 12 and one cover member 13b is removed. After mounting an injecting tool integrated with an injection needle, or mounting a connector capable of attaching a commercially available injection needle and attaching an injection needle thereto, another cover member 13b is removed and an injection piston member (extruding member) is inserted. Thus, it can be used as a kneaded cement mixture injector.

By filling the sterilized vessel with aforementioned powder and liquid, simultaneously revolving and rotating the vessel and kneading a material to be kneaded, a centrifugal force is applied to the material to be kneaded in the vessel. Consequently, the material to be kneaded is pressed against the inner wall of the vessel by the centrifugal force and the bubbles in the material to be kneaded are released by the pressure (deaeration), while the material to be kneaded in the vessel is agitated by the rotation of the vessel, thus making it possible to conduct strong and efficient kneading and to prepare a kneaded cement mixture having a high viscosity at a high powder-liquid ratio.

In case the material to be kneaded, which is composed of the powder and the liquid, is kneaded by simultaneously revolving and rotating the vessel, kneading is preferably conducted in the state where the vessel is inclined by 20 to 50 degrees. By inclining the vessel, a portion of the pressure due to the centrifugal force is applied to the material to be kneaded, thereby to accelerate agitation and deaeration of the material to be kneaded, along with a force produced by rotation of the vessel. When the inclination is less than 20 degrees, it becomes impossible to obtain satisfactory effect of accelerating agitation and deaeration of the material to be kneaded. On the other hand, when the inclination is greater than 50 degrees, it becomes impossible to obtain satisfactory effect of accelerating agitation and deaeration of the material to be kneaded and the contents are likely to jump out. Therefore, it is not preferred.

In the case of kneading the material to be kneaded, which is composed of the powder and the liquid, the vessel is preferably revolved at 1500 rpm or more and rotated at 300 rpm or more. When the revolution speed and rotation speed are lower than the aforementioned values, the agitation action and the deaeration action of the material to be kneaded become unsatisfactory, causing problems in that a uniform kneaded mixture cannot be obtained and the kneading time required to obtain the uniform kneaded mixture is prolonged.

When using the powder having a specific surface area within a range from 2.0 to 4.0 m²/g, the kneaded cement mixture of the present invention is prepared by kneading at a weight mixing ratio of the powder to the liquid (P/L) within a range from 1.5 to 3.5. When using the powder having a specific surface area within a range from 0.3 to 2.0 m²/g, the kneaded cement mixture is prepared by kneading at a weight mixing ratio of the powder to the liquid (P/L) within a range from 3.0 to 5.0.

When P/L is less than the lower limit value thereof, kneading can be conducted without using the kneading process of the present invention, and therefore there is no noticeable advantage except that the sterilized condition can be maintained. On the other hand, when P/L exceeds the upper limit value thereof, it is difficult to obtain a uniform kneaded mixture even by the kneading process of the present invention. When P/L is within a range from 2.0 to 3.0 (when using a powder having a surface area within a range from 2.0 to 4.0 m²/g) or when P/L is within a range from 3.5 to 4.5 (when using a powder having a surface area within a range from 0.3 to 2.0 m²/g), since the powder-liquid ratio is not achieved by a manual kneading process and the resulting kneaded cement mixture has fluidity sufficient to discharge it through a needle having an inner diameter of 2.0 mm, the maximum advantage of this process can be obtained.

Furthermore, the kneaded cement mixture of the present invention has a consistency within a range from 18 to 22 mm. The consistency of the kneaded mixture is measured by the following process. In the case of the powder having a specific surface area within a range from 2.0 to 4.0 m²/g (or 0.3 to 2.0 m²/g), 1 g of a cement paste prepared by kneading at P/L of 2.0 (or 3.5) is placed on a glass plate, and then a glass plate having a dead weight of 120 g is gently laid thereon. In that case, the length of the shortest axis and that of the longest axis of the spread cement paste are measured and an average value (unit: mm) is determined. When the consistency is greater than 22 mm, it becomes impossible to obtain a hardened cement material having a high strength. On the other hand, when the consistency is less than 18 mm, kneading is too difficult to obtain a uniform kneaded mixture and, furthermore, it becomes difficult to handle because of lack of fluidity.

In the kneaded cement mixture of the present invention, the amount of bubbles is preferably 10% or less based on the volume of the kneaded mixture. This bubble percentage is represented by percentage by volume of bubbles in the kneaded mixture or hardened material having a given volume. When the amount of bubbles is greater than 10%, the strength of the hardened material is lowered. Therefore, it is not preferred. In the case of kneading by the process of the present invention, it is made possible to obtain a kneaded cement mixture having a very small bubble percentage.

The hardened cement material provided by the present invention is solidified after filling the defective portion of bone with the resulting kneaded cement mixture thus obtained, or filling with the kneaded cement mixture in fixation and restoration of the bone fracture portion, or injecting it into a mold in which a cavity having a required shape and a required size is formed. This hardened cement material has a very high compression strength of 60 MPa or more.

### Second Example

In the second example of the present invention, the powder composition contains a calcium phosphate powder and a magnesium-containing compound. More preferably, it is substantially composed only of them. The other constituents may be the same as in the first example. The magnesium-containing compound used in this example can be at least one selected from magnesium salts such as phosphate, sulfate, carbonate and organic acid salt of magnesium; double salts with calcium; and compounds such as magnesium hydroxide and magnesium oxide. Particularly preferably, at least one of magnesium phosphate and magnesium sulfate can be used.

The magnesium-containing compound is incorporated to improve the fluidity of the calcium phosphate powder. Since an improvement in fluidity makes it difficult to form bubbles in the preparation of the hardened material, a biomedical cement having a higher strength can be obtained by adding the magnesium-containing compound.

Examples of the powder composition used in the second example include those prepared by mixing 99.97 to 50% by weight, preferably 95 to 60% by weight, and most preferably 90 to 70% by weight, of at least one of α-type tricalcium phosphate and tetracalcium phosphate, 1 to 49.97% by weight, and preferably 5 to 20% by weight, of dicalcium phosphate, 0 to 49.97% by weight, and preferably 3 to 15% by weight, of monocalcium phosphate, and 0.03 to 0.5% by weight of at least one of magnesium phosphate and magnesium sulfate. Those further containing 2 to 10% by weight, and preferably 3 to 7% by weight, of a hydrate of dicalcium phosphate and tricalcium phosphate as the arbitrary component may be used. The composition ratio, the firing temperature and the average particle diameter of the powder composition comprising the calcium phosphate powder and the magnesium-containing compound can be appropriately selected before use, taking into account the biocompatibility and purposes. The specific surface area, especially the specific surface area of the calcium phosphate powder, is decided within a certain range depending on the content thereof. In the present invention, the calcium phosphate powder composition having a specific surface area of 0.3 to 2.0 m²/g, or of 2.0 to 4.0 m²/g, is preferably used. The reason is as described above.

In the case in which the composition powder is prepared by mixing the magnesium-containing compound with the calcium phosphate powder, a predetermined amount of the magnesium-containing compound and a predetermined amount of the calcium phosphate powder are uniformly mixed using a appropriate mixing process such as dry-mixing, wet-mixing or grind-mixing process to give a composition powder. Alternatively, when the magnesium-containing compound is a soluble salt, a solution thereof is sprayed over the calcium phosphate powder and drying the powder to prepare a composition powder wherein the surface of the calcium phosphate powder is coated with the magnesium-containing compound. The hardening liquid and the kneading process used in this example may be the same as those described above.

### Third Example

In the third example of the present invention, the powder composition contains a calcium phosphate powder and a pyrophosphate.

As the pyrophosphate, at least one selected from the group consisting of an alkali metal salt, an alkali earth metal salt and an ammonium salt of pyrophosphoric acid can be used. Preferably, at least one of sodium pyrophosphate and potassium pyrophosphate is used. The pyrophosphate is incorporated to improve the fluidity of the calcium phosphate powder. Since an improvement in fluidity makes it difficult to form bubbles in the preparation of the hardened material, the addition of the pyrophosphate is effective to obtain a biomedical cement having a higher strength.

The powder composition, used in the third example, can contain a magnesium-containing compound, in addition to the calcium phosphate powder and the pyrophosphate described above. Similar to the second example, the magnesium-containing compound can be at least one selected from magnesium salts such as phosphate, sulfate, carbonate and organic acid salt of magnesium; double salts with calcium; and compounds such as magnesium hydroxide and magnesium oxide. Particularly preferably, at least one of magnesium phosphate and magnesium sulfate can be used. Similar to the pyrophosphate, this magnesium-containing compound exerts an effect of improving the fluidity of the calcium phosphate powder to yield a hardened biomedical cement material having a higher strength.

Examples of the powder composition used in this example include those prepared by mixing 100 parts by weight of a calcium phosphate powder containing 99.97 to 50% by weight, preferably 95 to 60% by weight, and most preferably 90 to 70% by weight, of at least one of α-type tricalcium phosphate and tetracalcium phosphate, 1 to 49.97% by weight, and preferably 5 to 20% by weight, of dicalcium phosphate, and 0 to 49.97% by weight, and preferably 3 to 15% by weight, of monocalcium phosphate with 0.03 to 0.5% by weight of a pyrophosphate. Those further containing 2 to 10% by weight, and preferably 3 to 7% by weight, of a hydrate of dicalcium phosphate and tricalcium phosphate as the arbitrary component may be used. The composition ratio, the firing temperature and the average particle diameter of the powder composition containing the calcium phosphate powder and the pyrophosphate can be appropriately selected before use, taking into account the biocompatibility and purposes. The specific surface area, especially the specific surface area of the calcium phosphate powder, is decided within a certain range depending on the content thereof. In the present invention, the calcium phosphate powder composition having a specific surface area of 0.3 to 2.0 m²/g, or of 2.0 to 4.0 m²/g, is preferably used.

The specific surface area is an important parameter having a strong relationship with the particle size, the reactivity and the fluidity of the calcium phosphate powder composition. When the specific surface area is less than 0.3 m²/g, the hardening time is prolonged because of poor reactivity, and the resulting product is not suited for use as a biomedical cement. On the other hand, when the specific surface area exceeds 4.0 m²/g, the fluidity of the resulting kneaded paste is reduced because of small particle size and high reactivity, and therefore a weight mixing ratio P/L of the powder to the liquid cannot be increased to a range from 1.5 to 5.0.

In the case in which the composition powder is prepared by mixing the pyrophosphate with the calcium phosphate powder, a predetermined amount of the pyrophosphate and a predetermined amount of the calcium phosphate powder are uniformly mixed using an appropriate mixing process such as dry-mixing, wet-mixing or grind-mixing process to yield a composition powder. Alternatively, an aqueous solution of the pyrophosphate is sprayed over the calcium phosphate powder and drying the powder to prepare a composition powder wherein the surface of the calcium phosphate powder is coated with the pyrophosphate.

### Test Examples

The following test examples clarify effects of the present invention, and the following test examples are therefore to be considered as illustrative and not restrictive. [Test with respect to a powder composition having a specific surface area within a range from 2.0 to 4.0 m²/g]

### Synthesis Example 1: α-type tricalcium phosphate

Calcium hydroxide (3 mol, manufactured by Ube Material Industries, Ltd.) was suspended in 10 liters of water and an aqueous 40 wt% solution prepared by diluting phosphoric acid (2 mol, manufactured by RASA Industries, LTD.) with water was gradually added dropwise to the suspension. After the completion of the dropwise addition, the mixture was allowed to stand at room temperature for one day and was dried in a dryer (manufactured by Yamato Science Co., Ltd. under the trade name of "Model DV61") at 110°C for 24 hours. The resulting aggregate was fired in an oven (manufactured by Motoyama Co., Ltd. under the trade name of "Model SuperBurn 2035") at 1250°C for 3 hours. Finally, the fired aggregate was ground by a grinder (manufactured by Ishikawa Kojo Co., Ltd. under the trade name of "Agitating Grinder, Vertical Type No. 20") and was then passed through a sieve having a mesh size of 88 µm to obtain α-type tricalcium phosphate having an average particle diameter of 4.5 µm.

### Synthesis Example 2: Tetracalcium phosphate

Using 4 mol of calcium hydroxide and 2 mol of phosphoric acid, the mixture was dried in the same manner as in Synthesis Example 1 to obtain an aggregate. The resulting aggregate was calcined at 900°C for 3 hours, taken out from an oven, uniformly ground, and then fired at 1400°C for 3 hours. Finally, the fired aggregate was ground by a grinder (manufactured by Ishikawa Kojo Co., Ltd. under the trade name of "Agitating Grinder, Vertical Type No. 20") and was then passed through a sieve having a mesh size of 88 µm to obtain tetracalcium phosphate having an average particle diameter of 6.0 µm.

### Test Example 1

95% of α-type tricalcium phosphate synthesized in Synthesis Example 1 was mixed with 5% of commercially available dicalcium phosphate (manufactured by Wako Pure Chemicals Industries, Ltd., reagent grade) to obtain a calcium phosphate powder composition. The specific surface area was measured by MONOSORB, Model MS-13, manufactured by Yuasa Ionics Co., Ltd. As a result, it was 2.38 m²/g.

An aqueous solution, as a hardening liquid, was prepared by dissolving 20% by weight of disodium succinate hexahydrate (manufactured by JUNSEI CHEMICAL Co., Ltd.) and 5% by weight of sodium chondroitin sulfate (manufactured by SEIKAGAKU CORPORATION, injection grade ND product).

10 g of the calcium phosphate powder composition was mixed with the liquid at a powder-liquid ratio (P/L) of 1.5, 2.0, 2.5, 3.0, 3.5 or 4.0, and then the mixture was charged in a vessel for exclusive use of "AWATORI RENTARO" (trade name, manufactured by THINKY Corp., specification: revolution speed of 2000 rpm and rotation speed of 400 rpm) and kneaded for 15 seconds. The results of the description test, the discharge test and the hardening time of the resulting kneaded cement mixture are shown in Table 1.

### Comparative Example 1

For comparison, the same powder and liquid as those used in Test Example 1 were charged in a commercially available mortar for medical use and then manually kneaded using a pestle. One minute was required to obtain a uniform paste. For comparison with Test Example 1, the results of the description test, the discharge test and the hardening time are shown in Table 1.

**TABLE 1**

| Powder-liquid ratio | | 1.5 | 2.0 | 2.5 | 3.0 | 3.5 | 4.0 |
|---|---|---|---|---|---|---|---|
| Test Example 1 | Description test | ○ | ○ | ○ | ○ | Δ | × |
| | Discharge test | ○ | ○ | ○ | ○ | × | × |
| | Hardening time | 6.5 mm. | 5.5 min. | 5.0 min. | 4.0 min. | 4.0 min. | - |
| Comparative Example 1 | Description test | ○ | ○ | Δ | × | × | × |
| | Discharge test | ○ | Δ | × | × | × | × |
| | Hardening time | 8.0 min. | 7.5 min. | 7.0 min. | - | - | - |

In the description test, the case where a completely uniform pasty or clay-like product is obtained was rated good "○", the case where nearly uniform product is obtained was rated ordinary "Δ", and the case where a uniform product is not obtained was rated poor "×". In the discharge test, the case where the product can be discharged through a needle having an inner diameter of 2 mm and a length of 40 mm was rated good "○", while the case where the product cannot be discharged through the needle was rated poor "×". This evaluation procedure is the same in the following tests.

### Test Example 2

### (Preparation of powder)

70.0% by weight of tetracalcium phosphate synthesized in Synthesis Example 2 was mixed with 30.0% by weight of commercially available dicalcium phosphate (manufactured by Wako Pure Chemicals Industries, Ltd., reagent grade) to obtain a calcium phosphate powder composition. The specific surface area was measured in the same manner as in Test Example 1. As a result, it was 3.48 m²/g.

### (Sterilization)

The powder was irradiated with γ-rays at a dose of 25 kGy and the liquid of Test Example 1 was also sterilized by filtration through a bacterial filter having a pore diameter of 0.22 µm (manufactured by NIHON PALL LTD.). A double vessel having a shape shown in Fig. 1 was prepared and an outer vessel (polycarbonate vessel, manufactured by Tochigi Seiko Co., Ltd.) was subjected to high pressure steam sterilization under the conditions of a temperature of 121 °C for 20 minutes, while an inner vessel (polypropylene vessel, manufactured by Tochigi Seiko Co., Ltd.) was sterilized in an ethylene oxide gas sterilizer (manufactured by Nitto Science Co., Ltd., under the trade name "Model CL-30-B").

### (Kneading)

The inner vessel was filled with 10 g of the powder and the liquid at P/L of 2.0, 2.5 or 3.0 and the inner vessel was set in the outer vessel. Then, the outer vessel was set in "SPINKLE" (trade name, manufactured by J. MORITA TOKYO MFG. CORP., specification: revolution speed of 2500 rpm and rotation speed of 500 rpm) and kneaded for 10 seconds.

### (Test)

The results of the hardening time and the strength test of the resulting kneaded cement mixture are shown in Table 2. The kneaded mixture was subjected to the "sterility test" in accordance with the general test procedure of the Japanese Pharmacopoeia. As a result, it was confirmed that the kneaded mixture is in the sterilized condition.

### Comparative Example 2

For comparison, the same powder and liquid as those used in Test Example 2 were charged at a powder-liquid ratio of 2.0 in a commercially available mortar for medical use and then manually kneaded for one minute, using a pestle. For comparison, the results of the hardening time and the compression strength are shown in Table 2.

**TABLE 2**

| | Comparative Example 2 | Test Example 2 | | |
|---|---|---|---|---|
| Powder-liquid ratio | 2.0 | 2.0 | 2.5 | 3.0 |
| Hardening time (min.) | 7.5 | 6.5 | 6.5 | 6.0 |
| Compression strength (MPa) | 45.2* | 55.0* | 58.8 | 62.5 |

| | | | | |
|---|---|---|---|---|
| *: There is a statistical significant difference in the results of the compression strength at a powder-liquid ratio of 2.0 of Comparative Example 2 and Test Example 2 (t-test, significance level: 5%). | | | | |

The compression strength and the hardening time were measured in accordance with JIS T6602 "Dental Zinc Phosphate Cement". The compression strength was measured in the wet condition after dipping a sample (7 mmφ × 14 mmL) in a simulated body fluid with the composition shown in Table 3 for 7 days. The loading speed was set to 0.5 mm/min. and a testing machine, Model 4455, manufactured by INSTRON CO.

**TABLE 3**

| (mM) | Na⁺ | K⁺ | Mg²⁺ | Ca²⁺ | Cl⁻ | HCO₃ | HPO₄²⁻ |
|---|---|---|---|---|---|---|---|
| Simulated body fluid | 142.0 | 5.0 | 1.5 | 2.5 | 148.8 | 4.2 | 1.0 |

As is apparent from the results shown in Table 1 and Table 2, the kneaded cement mixtures of Test Examples 1 and 2 prepared by the process of the present invention can be prepared within a shorter time as compared with the kneaded mixtures of Comparative Examples 1 and 2 prepared by a manual process (using a mortar) and the powder can be kneaded with the liquid at a high powder-liquid ratio. In Test Example 1, kneaded cement mixtures capable of being discharged through a needle could be prepared at the powder-liquid ratio within a range from 1.5 to 3.0.

As is apparent from the results of Table 2, the kneaded cement mixture of the Test Example 2 prepared by the process of the present invention achieves a higher compression strength on hardening as compared with the kneaded mixture of Comparative Example 2 prepared by a manual process. As described in Test Example 2, a kneaded cement mixture could be prepared in the sterilized condition using a double vessel.

In Test Examples 1 and 2, various kneaded cement mixtures were prepared by varying the powder-liquid ratio and the kneading conditions (revolution speed and rotation speed) and the results of the description test, the discharge test, the hardening time and the compression strength were compared with those of the kneaded mixtures of Comparative Examples 1 and 2 prepared by a manual process (mortar). The results are summarized in Table 4 to Table 7.

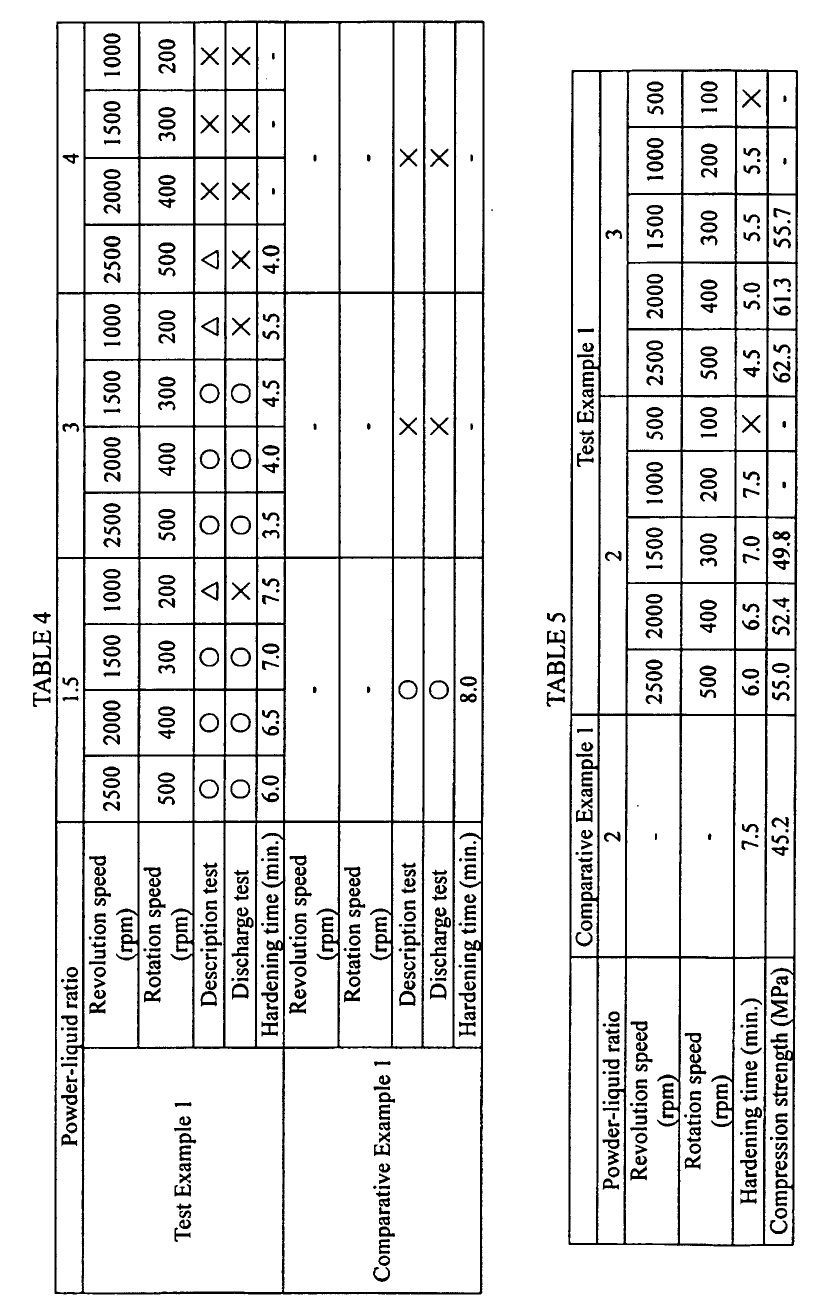

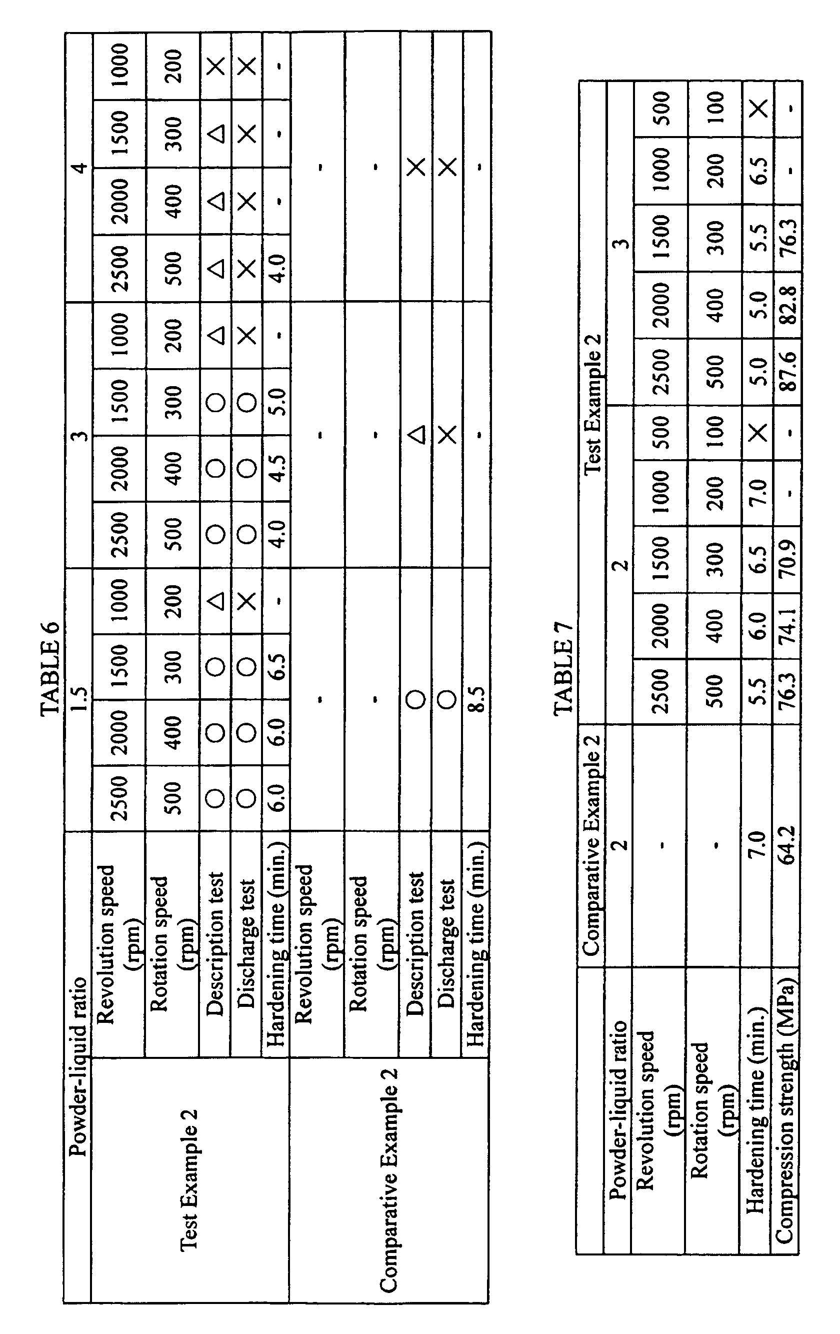

### [Test with respect to a powder composition having a specific surface area within a range from 0.3 to 2.0 m²/g]

### Synthesis Example 3: α-type tricalcium phosphate

Calcium hydroxide (3 mol, manufactured by Ube Material Industries, Ltd.) was suspended in 10 liters of water and an aqueous 40 wt% solution prepared by diluting phosphoric acid (2 mol, manufactured by RASA Industries, LTD.) with water was gradually added dropwise to the suspension. After the completion of the dropwise addition, the mixture was allowed to stand at room temperature for one day and was dried in a dryer (manufactured by Yamato Science Co., Ltd. under the trade name of "Model DV61") at 110°C for 24 hours. The resulting aggregate was fired in an oven (manufactured by Motoyama Co., Ltd. under the trade name of "Model SuperBurn 2035") at 1400°C for 3 hours. Finally, the fired aggregate was ground by a grinder (manufactured by Ishikawa Kojo Co., Ltd. under the trade name of "Agitating Grinder, Vertical Type No. 20") and then passed through a sieve having a mesh size of 88 µm to obtain α-type tricalcium phosphate having an average particle diameter of 6.50 µm.

### Synthesis Example 4: Tetracalcium phosphate

Using 4 mol of calcium hydroxide and 2 mol of phosphoric acid, the mixture was dried in the same manner as in Synthesis Example 1 to obtain an aggregate. The resulting aggregate was calcined at 900°C for 3 hours, taken out of the oven, uniformly ground, and then fired at 1400°C for 3 hours. Finally, the fired aggregate was ground by a grinder (manufactured by Ishikawa Kojo Co., Ltd. under the trade name of "Agitating Grinder, Vertical Type No. 20") and then passed through a sieve having a mesh size of 88 µm to obtain tetracalcium phosphate having an average particle diameter of 6.0 µm.

### Test Example 3

95% of α-type tricalcium phosphate synthesized in Synthesis Example 3 was mixed with 5% of commercially available dicalcium phosphate (manufactured by Wako Pure Chemicals Industries, Ltd., reagent grade) to obtain a calcium phosphate powder composition. The specific surface area was measured by MONOSORB, Model MS-13, manufactured by Yuasa Ionics Co., Ltd. As a result, it was 0.82 m²/g.

An aqueous solution, as a hardening liquid, was prepared by dissolving 20% by weight of disodium succinate hexahydrate (manufactured by JUNSEI CHEMICAL Co., Ltd.) and 5% by weight of sodium chondroitin sulfate (manufactured by SEIKAGAKU CORPORATION, injection grade ND product).

10 g of the calcium phosphate powder composition was mixed with the liquid at a powder-liquid ratio (P/L) of 3.0, 3.5, 4.0, 4.5, 5.0 or 5.5 and then the mixture was charged in a vessel for exclusive use of "AWATORI RENTARO" (trade name, manufactured by THINKY Corp., specification: revolution speed of 2000 rpm and rotation speed of 400 rpm) and kneaded for 15 seconds. The results of the description test, the discharge test and the hardening time of the resulting kneaded cement mixture are shown in Table 8.

### Comparative Example 3

For comparison, the same powder and liquid as those used in Test Example 3 were charged in a commercially available mortar for medical use and then manually kneaded using a pestle. One minute was required to obtain a uniform paste. For comparison with Test Example 3, the results of the description test, the discharge test and the hardening time are shown in Table 8.

**TABLE 8**

| Powder-liquid ratio | | 3.0 | 3.5 | 4.0 | 4.5 | 5.0 | 5.5 |
|---|---|---|---|---|---|---|---|
| Test Example 3 | Description test | ○ | ○ | ○ | ○ | Δ | × |
| | Discharge test | ○ | ○ | ○ | ○ | × | × |
| | Hardening time | 6.0 min. | 5.0 min. | 4.5 min. | 4.0 min. | 3.5 min. | - |
| Comparative Example 3 | Description test | ○ | Δ | × | × | × | × |
| | Discharge test | Δ | × | × | × | × | × |
| | Hardening time | 8.0 min. | 7.5 min. | - | - | - | - |

### Test Example 4

### (Preparation of powder)

75.0% by weight of α-type tricalcium phosphate synthesized in Synthesis Example 1, 20.0% by weight of tetracalcium phosphate of Synthesis Example 2 and 5.0% by weight of commercially available dicalcium phosphate (manufactured by Wako Pure Chemicals Industries, Ltd., reagent grade) were mixed to obtain a calcium phosphate powder composition. The specific surface area was measured in the same manner as in Test Example 1. As a result, it was 1.27 m²/g.

### (Sterilization)

The powder was irradiated with γ-rays at a dose of 25 kGy and the liquid of Test Example 3 was also sterilized by filtration through a bacterial filter having a pore diameter of 0.22 µm (manufactured by NIHON PALL LTD.). A double vessel having a shape shown in Fig. 1 was prepared and an outer vessel (polycarbonate vessel, manufactured by Tochigi Seiko Co., Ltd.) was subjected to high pressure steam sterilization under the conditions of a temperature of 121°C for 20 minutes, while an inner vessel (polypropylene vessel, manufactured by Tochigi Seiko Co., Ltd.) was sterilized in an ethylene oxide gas sterilizer (manufactured by Nitto Science Co., Ltd. under the trade name of "Model CL-30-B").

### (Kneading)

The inner vessel was filled with 10 g of the powder and the liquid at P/L of 3.0, 3.5 or 4.0 and the inner vessel was set in the outer vessel. Then, the outer vessel was set in "SPINKLE" (trade name, manufactured by J.MORITA TOKYO MFG. CORP., specification: revolution speed of 2500 rpm and rotation speed of 500 rpm) and kneaded for 10 seconds.

### (Test)

The results of the hardening time and the strength test of the resulting kneaded cement mixture are shown in Table 9. The kneaded mixture was subjected to the "sterility test" in accordance with the general test procedure of the Japanese Pharmacopoeia. As a result, it was confirmed that the kneaded mixture is in the sterilized condition.

### Comparative Example 4

For comparison, the same powder and liquid as those used in Test Example 4 were charged at a powder-liquid ratio of 2.0 in a commercially available mortar for medical use and then manually kneaded for one minute, using a pestle. For comparison, the results of the hardening time and the compression strength are shown in Table 9.

**TABLE 9**

| | Comparative Example 4 | Test Example 4 | | |
|---|---|---|---|---|
| Powder-liquid ratio | 3.0 | 3.0 | 3.5 | 4.0 |
| Hardening time (min.) | 6.5 | 5.5 | 5.0 | 5.0 |
| Compression strength (MPa) | 67.2* | 74.4* | 88.2 | 94.7 |

As is apparent from the results shown in Table 8 and Table 9, the kneaded cement mixtures of Test Examples 3 and 4 prepared by the process of the present invention can be prepared within a shorter time as compared with the kneaded mixtures of Comparative Examples 3 and 4 prepared by a manual process (using a mortar) and the powder can be kneaded with the liquid at a high powder-liquid ratio. In Test Example 3, kneaded cement mixtures capable of being discharged through a needle could be prepared at the powder-liquid ratio within a range from 3.0 to 4.5. As is apparent from the results of Table 9, the kneaded cement mixture of the Test Example 4 prepared by the process of the present invention achieves a higher compression strength on hardening as compared with the kneaded mixture of Comparative Example 4 prepared by a manual process. As described in Test Example 4, a kneaded cement mixture could be prepared in the sterilized condition using a double vessel.

In Test Examples 3 and 4, various kneaded cement mixtures were prepared by varying the powder-liquid ratio and the kneading conditions (revolution speed and rotation speed) and the results of the description test, the discharge test, the hardening time and the compression strength were compared with those of the kneaded mixtures of Comparative Examples 3 and 4 prepared by a manual process (mortar). The results are summarized in Table 10 to Table 13.

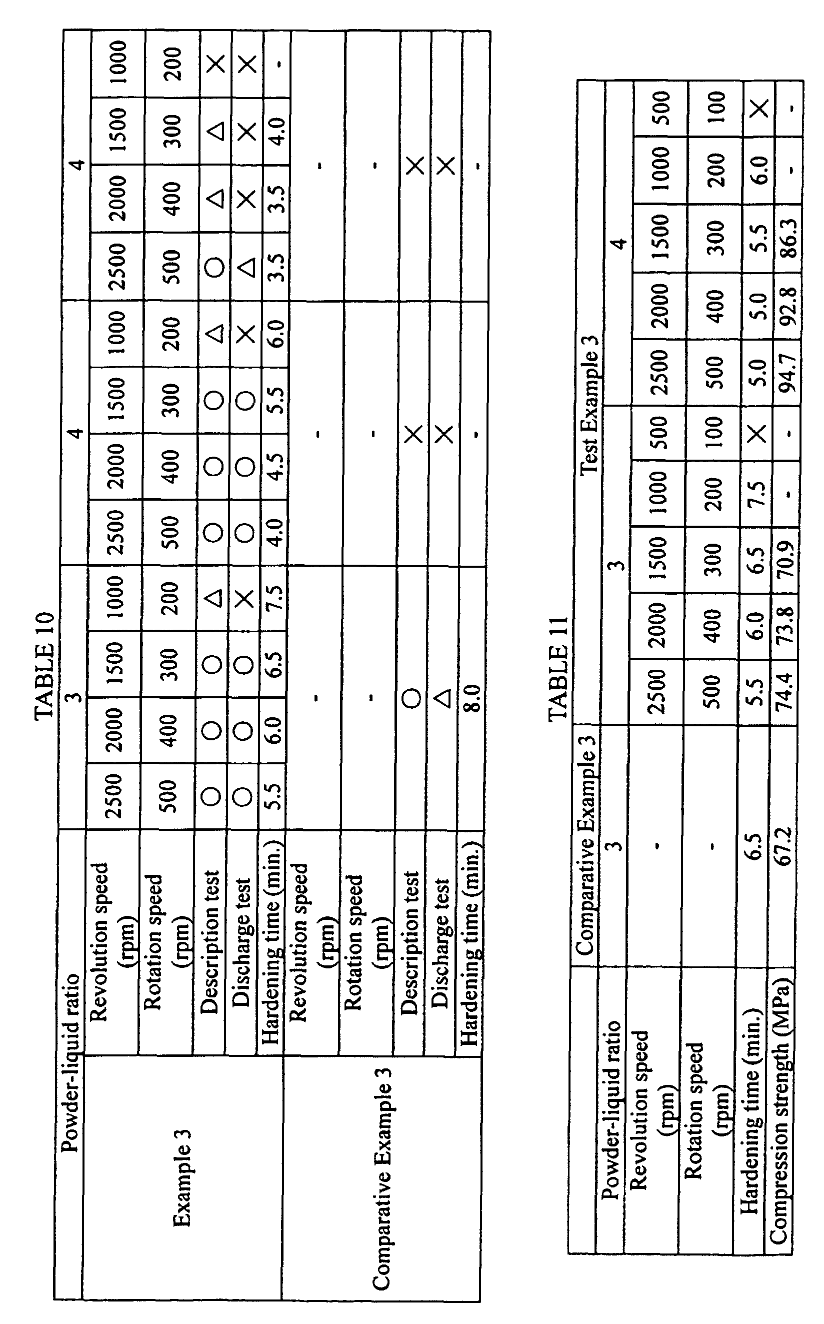

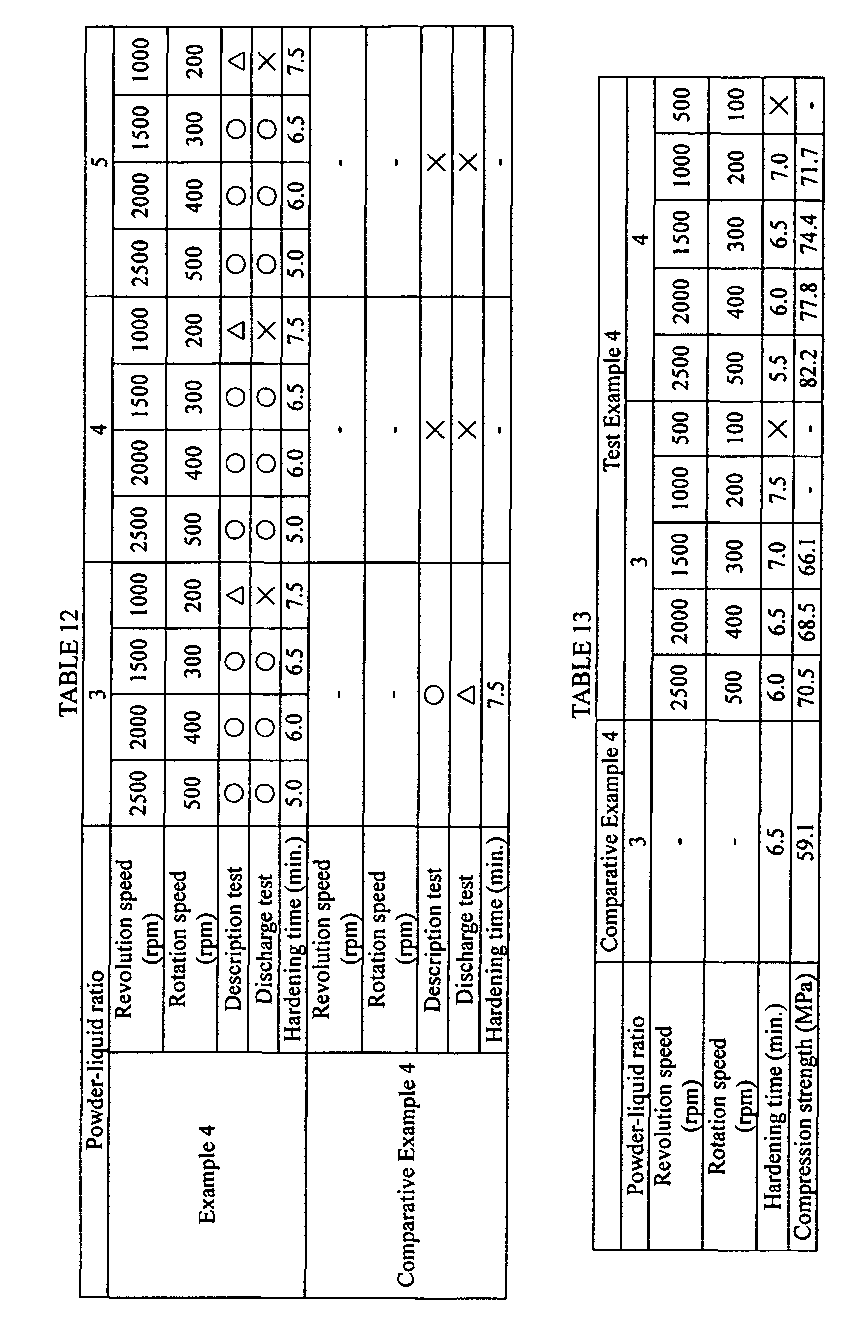

### [Test with respect to a powder composition which contains a magnesium-containing compound and has a specific surface area within a range from 2.0 to 4.0 m²/g]

### Test Example 5

95% of α-type tricalcium phosphate synthesized in Synthesis Example 1 was mixed with 5% of commercially available dicalcium phosphate (manufactured by Wako Pure Chemicals Industries, Ltd., reagent grade) to obtain a calcium phosphate powder. The specific surface area was measured by MONOSORB, Model MS-13, manufactured by Yuasa Ionics Co., Ltd. As a result, it was 2.38 m²/g. To 100 parts by weight of the calcium phosphate powder, 0.2 parts by weight of magnesium phosphate (manufactured by KANTO KAGAKU) was added, followed by mixing using a grinder (manufactured by Ishikawa Kojo Co., Ltd.) to give a powder composition.

An aqueous solution, as a hardening liquid, was prepared by dissolving 20% by weight of disodium succinate hexahydrate (manufactured by JUNSEI CHEMICAL Co., Ltd.) and 5% by weight of sodium chondroitin sulfate (manufactured by SEIKAGAKU CORPORATION, injection grade ND product).

10 g of the powder composition was mixed with the liquid at a powder-liquid ratio (P/L) of 1.5, 2.0, 2.5, 3.0, 3.5 or 4.0 and then the mixture was charged in a vessel for exclusive use of "AWATORI RENTARO" (trade name, manufactured by THINKY Corp., specification: revolution speed of 2000 rpm and rotation speed of 400 rpm) and kneaded for 15 seconds. The results of the description test, the discharge test and the hardening time of the resulting kneaded cement mixture are shown in Table 14.

### Comparative Example 5

For comparison, the same powder and liquid as those used in Test Example 5 were charged in a commercially available mortar for medical use and then manually kneaded using a pestle. One minute was required to obtain a uniform paste. For comparison with Test Example 5, the results of the description test, the discharge test and the hardening time are shown in Table 14.

**TABLE 14**

| Powder-liquid ratio | | 1.5 | 2.0 | 2.5 | 3.0 | 3.5 | 4.0 |
|---|---|---|---|---|---|---|---|
| Test Example 5 | Description test | ○ | ○ | ○ | ○ | Δ | × |
| | Discharge test | ○ | ○ | ○ | ○ | × | × |
| | Hardening time | 7.0 min. | 6.0 min. | 5.0 min. | 4.5 min. | 4.0 min. | - |
| Comparative Example 5 | Description test | ○ | ○ | Δ | × | × | × |
| | Discharge test | ○ | Δ | × | × | × | × |
| | Hardening time | 8.5 min. | 8.0 min. | 7.5 min. | - | - | - |

### Test Example 6

### (Preparation of powder)

70.0% by weight of α-type tetracalcium phosphate synthesized in Synthesis Example 2 was mixed with 30.0% by weight of commercially available dicalcium phosphate (manufactured by Wako Pure Chemicals Industries, Ltd.) were mixed to obtain a calcium phosphate powder. To 100 parts by weight of the calcium phosphate powder, 0.4 parts by weight of magnesium phosphate having the same particle diameter (manufactured by KANTO KAGAKU) was added, followed by mixing to give a powder composition. The specific surface area of the powder composition was measured in the same manner as in Test Example 5. As a result, it was 3.48 m²/g.

### (Sterilization)

The powder composition was irradiated with γ-rays at a dose of 25 kGy and the liquid of Test Example 5 was also sterilized by filtration through a bacterial filter having a pore diameter of 0.22 µm (manufactured by NIHON PALL LTD.). A double vessel having a shape shown in Fig. 1 was prepared and an outer vessel (polycarbonate vessel, manufactured by Tochigi Seiko Co., Ltd.) was subjected to high pressure steam sterilization under the conditions of a temperature of 121°C for 20 minutes, while an inner vessel (polypropylene vessel, manufactured by Tochigi Seiko Co., Ltd.) was sterilized in an ethylene oxide gas sterilizer (manufactured by Nitto Science Co., Ltd. under the trade name of "Model CL-30-B").

### (Kneading)

The inner vessel was filled with 10 g of the powder and the liquid at P/L of 2.0, 2.5 or 3.0 and the inner vessel was set in the outer vessel. Then, the outer vessel was set in "SPINKLE" (trade name, manufactured by J. MORITA TOKYO MFG. CORP., specification: revolution speed of 2500 rpm and rotation speed of 500 rpm) and was kneaded for 10 seconds.

### (Test)

The results of the hardening time and the strength test of the resulting biomedical kneaded cement mixture are shown in Table 15. The kneaded mixture was subjected to the "sterility test" in accordance with the general test procedure of the Japanese Pharmacopoeia. As a result, it was confirmed that the kneaded mixture was in the sterilized condition.

### Comparative Example 6

For comparison, the same powder and liquid as those used in Test Example 6 were charged at a powder-liquid ratio of 2.0 in a commercially available mortar for medical use and then manually kneaded for one minute, using a pestle. For comparison, the results of the hardening time and the compression strength are shown in Table 15.

**TABLE 15**

| | Comparative Example 6 | Test Example 6 | | |
|---|---|---|---|---|
| Powder-liquid ratio | 2.0 | 2.0 | 2.5 | 3.0 |
| Hardening time (min.) | 8.0 | 7.0 | 6.0 | 5.5 |
| Compression strength (MPa) | 51.2* | 60.9* | 64.3 | 68.8 |

| | | | | |
|---|---|---|---|---|
| *: There is a statistical significant difference in the results of the compression strength at a powder-liquid ratio of 2.0 of Comparative Example 6 and Test Example 6 (t-test, significance level: 5%). | | | | |

The compression strength and the hardening time were measured in accordance with JIS T6602 "Dental Zinc Phosphate Cement". The compression strength was measured in the wet condition after dipping a sample (7 mm φ × 14 mmL) in a simulated body fluid with the composition shown in Table 3 for 7 days. The loading speed was set to 0.5 mm/min. and the testing machine was Model 4455, manufactured by INSTRON CO.

As is apparent from the results shown in Table 14 and Table 15, the biomedical kneaded cement mixtures of Test Examples 5 and 6 prepared by the process of the present invention can be prepared within a shorter time compared with the kneaded mixtures of Comparative Examples 5 and 6 prepared by a manual process (using a mortar) and the powder can be kneaded with the liquid at a high powder-liquid ratio. In Test Example 5, biomedical kneaded cement mixtures capable of being discharged through a needle could be prepared at the powder-liquid ratio within a range from 1.5 to 3.0. As is apparent from the results of Table 15, the biomedical kneaded cement mixture of Test Example 6 prepared by the process of the present invention achieves a higher compression strength on hardening as compared with the kneaded mixture of Comparative Example 6 prepared by a manual process. As described in Test Example 6, a biomedical kneaded cement mixture could be prepared in the sterilized condition using a double vessel.

In Test Examples 5 and 6, various biomedical kneaded cement mixtures were prepared by varying the powder-liquid ratio and the kneading conditions (revolution speed and rotation speed) and the results of the description test, the discharge test, the hardening time and the compression strength were compared with those of the kneaded mixtures of Comparative Examples 5 and 6 prepared by a manual process (mortar). The results are summarized in Table 16 to Table 19.

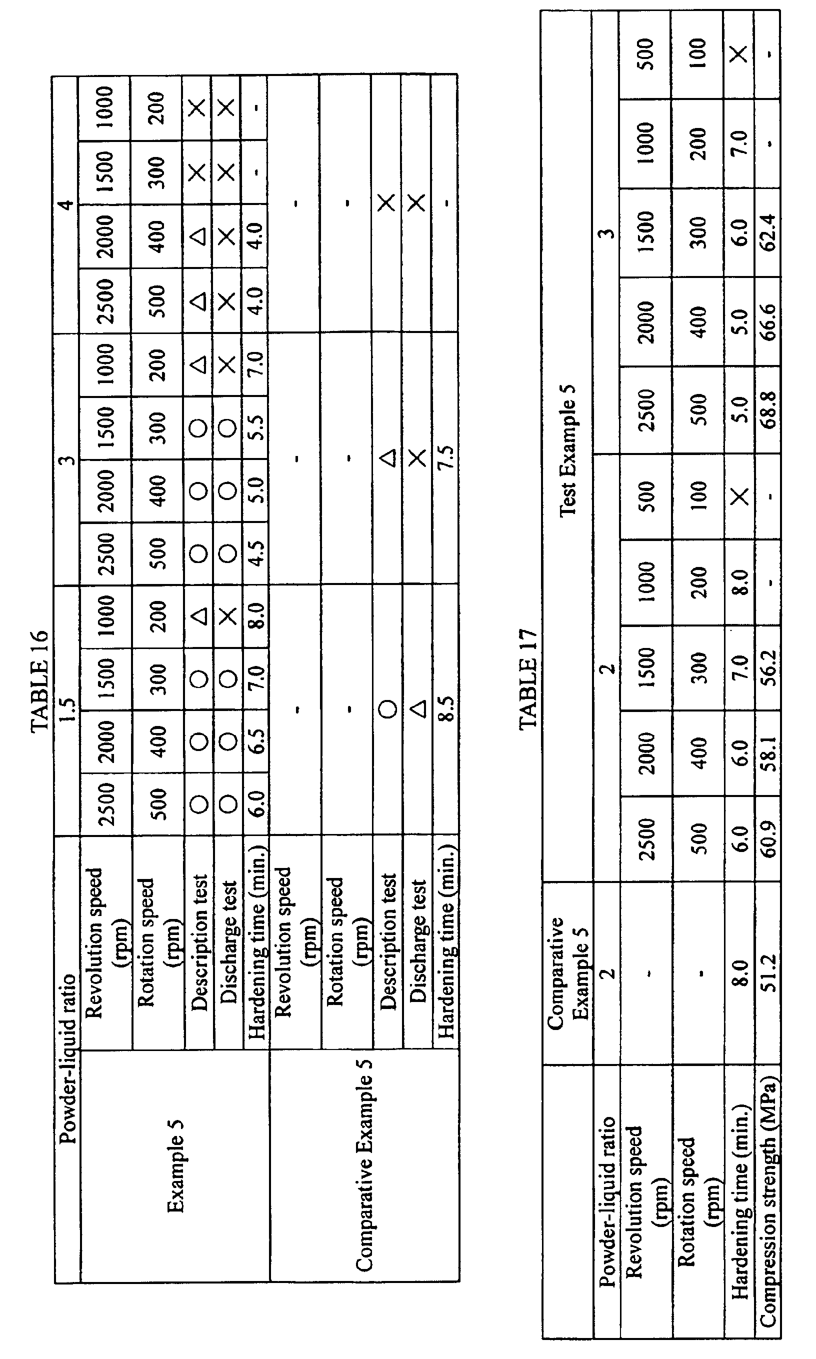

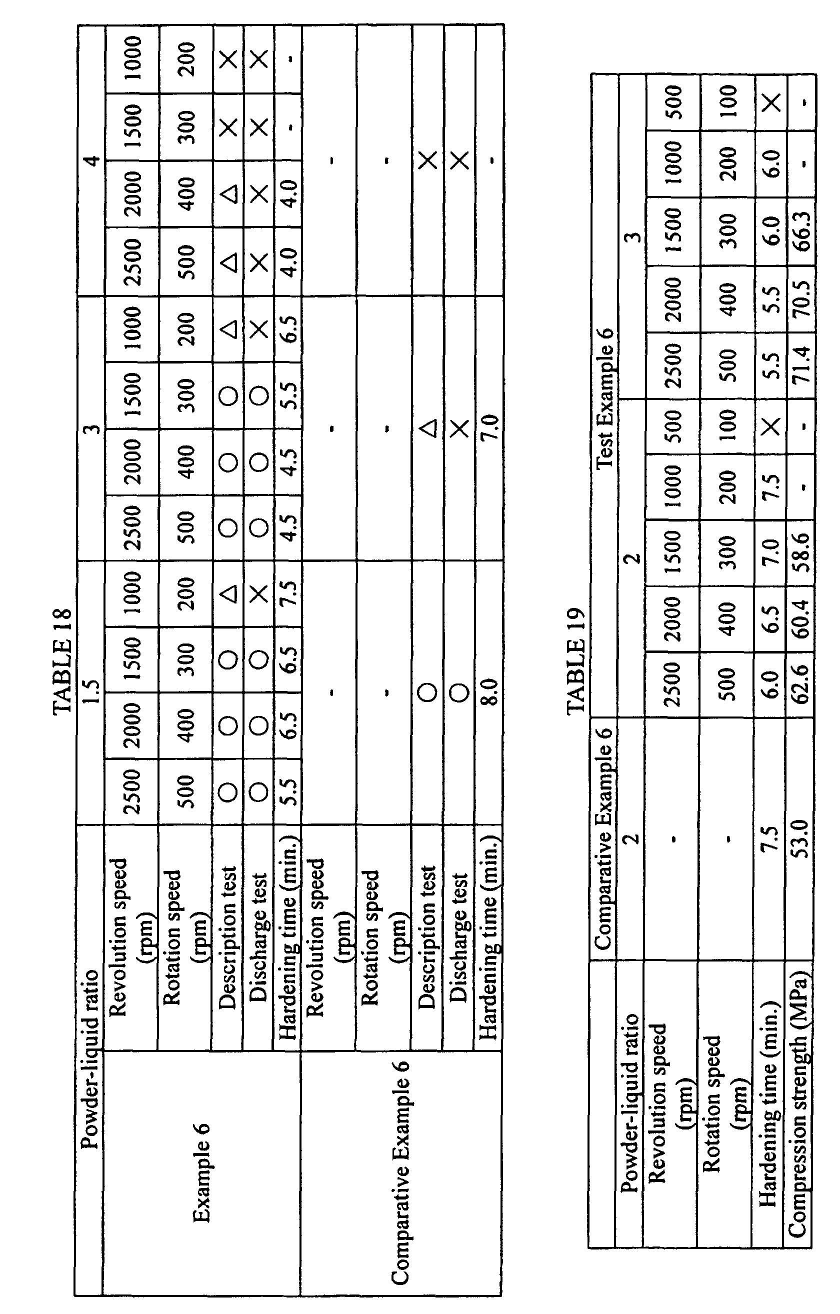

### [Test with respect to a powder composition having a specific surface area within a range from 0.3 to 2.0 m²/g]

### Test Example 7

95% of α-type tricalcium phosphate synthesized in Synthesis Example 3 was mixed with 5% of commercially available dicalcium phosphate (manufactured by Wako Pure Chemicals Industries, Ltd., reagent grade) to obtain a calcium phosphate powder. To 100 parts by weight of this calcium phosphate powder, 0.2 parts by weight of either of magnesium phosphate and magnesium sulfate (manufactured by KANTO KAGAKU) was added, followed by mixing using a grinder (manufactured by Ishikawa Kojo Co., Ltd.) to obtain a powder composition. The specific surface area of the resulting powder composition was measured by MONOSORB, Model MS-13, manufactured by Yuasa Ionics Co., Ltd. As a result, it was 0.82 m²/g.

An aqueous solution, as a hardening liquid, was prepared by dissolving 20% by weight of disodium succinate hexahydrate (manufactured by JUNSEI CHEMICAL Co., Ltd.) and 5% by weight of sodium chondroitin sulfate (manufactured by SEIKAGAKU CORPORATION, injection grade ND product).

10 g of the powder composition was mixed with the liquid at a powder-liquid ratio (P/L) of 3.0, 3.5, 4.0, 4.5, 5.0 or 5.5, and then the mixture was charged in a vessel for exclusive use of "AWATORI RENTARO" (trade name, manufactured by THINKY Corp., specification: revolution speed of 2000 rpm and rotation speed of 400 rpm) and was kneaded for 15 seconds. The results of the description test, the discharge test and the hardening time of the resulting biomedical kneaded cement mixture are shown in Table 20.

### Comparative Example 7

For comparison, the same powder and liquid as those used in Test Example 7 were charged in a commercially available mortar for medical use and then manually kneaded using a pestle. One minute was required to obtain a uniform paste. For comparison with Test Example 7, the results of the description test, the discharge test and the hardening time are shown in Table 20.

**TABLE 20**

| Powder-liquid ratio | | 3.0 | 3.5 | 4.0 | 4.5 | 5.0 | 5.5 |
|---|---|---|---|---|---|---|---|
| Test Example 7 | Description test | ○ | ○ | ○ | ○ | Δ | × |
| | Discharge test | ○ | ○ | ○ | ○ | × | × |
| | Hardening time | 6.5 min. | 5.5 min. | 5.0 min. | 4.5 min. | 4.0 min. | - |
| Comparative Example 7 | Description test | ○ | Δ | × | × | × | × |
| | Discharge test | Δ | × | × | × | × | × |
| | Hardening time | 8.5 min. | 7.5 min. | | - | - | - |

### Test Example 8

### (Preparation of powder)

75.0% by weight of α-type tricalcium phosphate synthesized in Synthesis Example 1, 20.0% by weight of tetracalcium phosphate of Synthesis Example 2 and 5.0% by weight of commercially available dicalcium phosphate (manufactured by Wako Pure Chemicals Industries, Ltd.) to obtain a calcium phosphate powder. To 100 parts by weight of this calcium phosphate powder, 0.2 parts by weight of either of magnesium phosphate and magnesium sulfate (manufactured by KANTO KAGAKU) was added, followed by mixing using a grinder (manufactured by Ishikawa Kojo Co., Ltd.) to obtain a powder composition. The specific surface area of the resulting powder composition was measured in the same manner as in Test Example 1. As a result, it was 1.27 m²/g.

### (Sterilization)

The powder was irradiated with γ-rays at a dose of 25 kGy and the liquid of Test Example 7 was also sterilized by filtration through a bacterial filter having a pore diameter of 0.22 µm (manufactured by NIHON PALL LTD.). A double vessel having a shape shown in Fig. 1 was prepared and an outer vessel (polycarbonate vessel, manufactured by Tochigi Seiko Co., Ltd.) was subjected to high pressure steam sterilization under the conditions of a temperature of 121 °C for 20 minutes, while an inner vessel (polypropylene vessel, manufactured by Tochigi Seiko Co., Ltd.) was sterilized in an ethylene oxide gas sterilizer (manufactured by Nitto Science Co., Ltd. under the trade name of "Model CL-30-B").

### (Kneading)

The inner vessel was filled with 10 g of the powder and the liquid at P/L of 3.0, 3.5 or 4.0 and the inner vessel was set in the outer vessel. Then, the outer vessel was set in a "SPINKLE" (trade name, manufactured by J. MORITA TOKYO MFG. CORP., specification: revolution speed of 2500 rpm and rotation speed of 500 rpm) and kneaded for 10 seconds.

### (Test)

The results of the hardening time and the strength test of the resulting biomedical kneaded cement mixture are shown in Table 21. The kneaded mixture was subjected to the "sterility test" in accordance with the general test procedure of the Japanese Pharmacopoeia. As a result, it was confirmed that the kneaded mixture was in the sterilized condition.

### Comparative Example 8

For comparison, the same powder and liquid as those used in Test Example 8 were charged at a powder-liquid ratio of 2.0 in a commercially available mortar for medical use and then manually kneaded for one minute, using a pestle. For comparison, the results of the hardening time and the compression strength are shown in Table 21.

**TABLE 21**

| | Comparative Example 8 | Test Example 8 | | |
|---|---|---|---|---|
| Powder-liquid ratio | 3.0 | 3.0 | 3.5 | 4.0 |
| Hardening time (min.) | 7.0 | 6.0 | 5.5 | 5.0 |
| Compression strength (MPa) | 74.8* | 84.5* | 95.2 | 100.4 |

| | | | | |
|---|---|---|---|---|
| *: There is a statistical significant difference in the results of the compression strength at a powder-liquid ratio of 3.0 of Comparative Example 8 and Test Example 8 (t-test, significance level: 5%). | | | | |

As is apparent from the results shown in Table 20 and Table 21, the biomedical kneaded cement mixtures of Test Examples 7 and 8 prepared by the process of the present invention can be prepared within a shorter time as compared with the kneaded mixtures of Comparative Examples 7 and 8 prepared by a manual process (using a mortar) and the powder can be kneaded with the liquid at a high powder-liquid ratio. In Test Example 7, biomedical kneaded cement mixtures capable of being discharged through a needle could be prepared at a powder-liquid ratio within a range from 3.0 to 4.5. As is apparent from the results of Table 21, the biomedical kneaded cement mixture of the Test Example 8 prepared by the process of the present invention achieves a higher compression strength on hardening as compared with the kneaded mixture of Comparative Example 8 prepared by a manual process. As described in Test Example 8, a biomedical kneaded cement mixture could be prepared in the sterilized condition using a double vessel.

In Test Examples 7 and 8, various biomedical kneaded cement mixtures were prepared by varying the powder-liquid ratio and the kneading conditions (revolution speed and rotation speed) and the results of the description test, the discharge test, the hardening time and the compression strength were compared with those of the kneaded mixtures of Comparative Examples 7 and 8 prepared by a manual process (mortar). The results are summarized in Table 22 to Table 25.

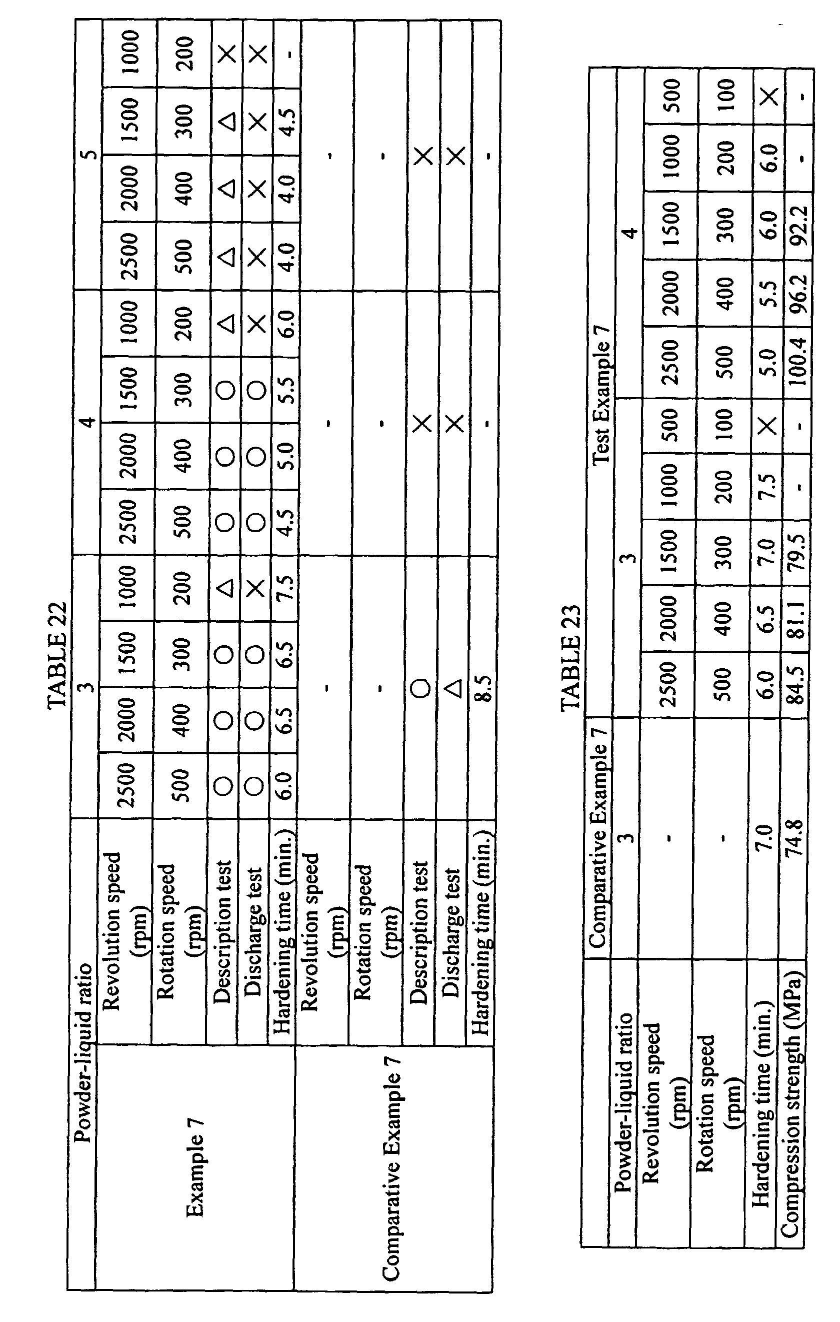

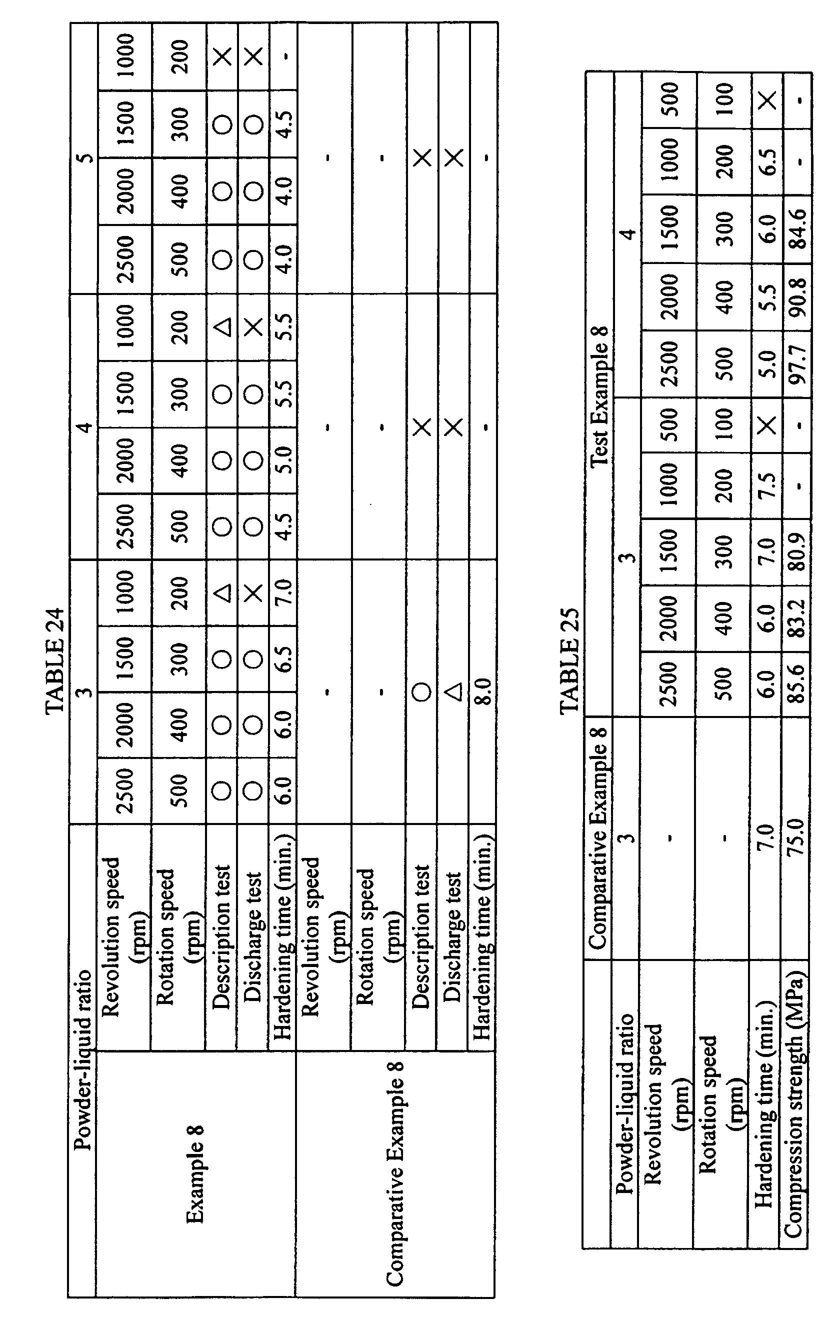

### [Test with respect to a powder composition which contains a pyrophosphate and has a specific surface area within a range from 2.0 to 4.0 m²/g]

### Test Example 9

95% of α-type tricalcium phosphate synthesized in Synthesis Example 1 was mixed with 5% of commercially available dicalcium phosphate (manufactured by Wako Pure Chemicals Industries, Ltd., reagent grade) to obtain a calcium phosphate powder. The specific surface area of the resulting calcium phosphate powder was measured by MONOSORB, Model MS-13, manufactured by Yuasa Ionics Co., Ltd. As a result, it was 2.38 m²/g. To 100 parts by weight of this calcium phosphate powder, 0.2 parts by weight of sodium pyrophosphate (manufactured by JUNSEI CHEMICAL Co., Ltd.) was added, followed by mixing using a grinder (manufactured by Ishikawa Kojo Co., Ltd.) to obtain a powder composition.

An aqueous solution, as a hardening liquid, was prepared by dissolving 20% by weight of disodium succinate hexahydrate (manufactured by JUNSEI CHEMICAL Co., Ltd.) and 5% by weight of sodium chondroitin sulfate (manufactured by SEIKAGAKU CORPORATION, injection grade ND product).

10 g of the powder composition was mixed with the liquid at a powder-liquid ratio (P/L) of 1.5, 2.0, 2.5, 3.0, 3.5 or 4.0 and then the mixture was charged in a vessel for exclusive use of "AWATORI RENTARO" (trade name, manufactured by THINKY Corp., specification: revolution speed of 2000 rpm and rotation speed of 400 rpm) and kneaded for 15 seconds. The results of the description test, the discharge test and the hardening time of the resulting biomedical kneaded cement mixture are shown in Table 26.

### Comparative Example 9

For comparison, the same powder and liquid as those used in Test Example 9 were charged in a commercially available mortar for medical use and then manually kneaded using a pestle. One minute was required to obtain a uniform paste. For comparison with Test Example 9, the results of the description test, the discharge test and the hardening time are shown in Table 26.

**TABLE 26**

| Powder-liquid ratio | | 1.5 | 2.0 | 2.5 | 3.0 | 3.5 | 4.0 |
|---|---|---|---|---|---|---|---|
| Test Example 9 | Description test | ○ | ○ | ○ | ○ | Δ | × |
| | Discharge test | ○ | ○ | ○ | ○ | × | × |
| | Hardening time | 7.0 min. | 6.5 min. | 5.0 min. | 4.5 min. | 4.5 min. | - |
| Comparative Example 9 | Description test | ○ | ○ | Δ | × | × | × |
| | Discharge test | ○ | Δ | × | × | × | × |
| | Hardening time | 9.0 min. | 8.0 min. | 7.5 min. | - | - | - |

### Test Example 10

### (Preparation of powder)

70.0% by weight of tetracalcium phosphate synthesized in Synthesis Example 2 was mixed with 30.0% by weight of commercially available dicalcium phosphate (manufactured by Wako Pure Chemicals Industries, Ltd.) were mixed to obtain a calcium phosphate powder. To 100 parts by weight of the calcium phosphate powder, 0.4 parts by weight of sodium pyrophosphate (manufactured by JUNSEI CHEMICAL Co., Ltd.) was added, followed by mixing to yeild a powder composition. The specific surface area of the powder composition was measured in the same manner as in Test Example 9. As a result, it was 3.48 m²/g.

### (Sterilization)

The powder composition was irradiated with γ-rays at a dose of 25 kGy and the liquid of Test Example 9 was also sterilized by filtration through a bacterial filter having a pore diameter of 0.22 µm (manufactured by NIHON PALL LTD.). A double vessel having a shape shown in Fig. 1 was prepared and an outer vessel (polycarbonate vessel, manufactured by Tochigi Seiko Co., Ltd.) was subjected to high pressure steam sterilization under the conditions of a temperature of 121 °C for 20 minutes, while an inner vessel (polypropylene vessel, manufactured by Tochigi Seiko Co., Ltd.) was sterilized in an ethylene oxide gas sterilizer (manufactured by Nitto Science Co., Ltd., under the trade name of "Model CL-30-B").

### (Kneading)

The inner vessel was filled with 10 g of the powder and the liquid at P/L of 2.0, 2.5, or 3.0 and the inner vessel was set in the outer vessel. Then, the outer vessel was set in a "SPINKLE" (trade name, manufactured by J. MORITA TOKYO MFG. CORP., specification: revolution speed of 2500 rpm and rotation speed of 500 rpm) and kneaded for 10 seconds.

### (Test)

The results of the hardening time and the strength test of the resulting biomedical kneaded cement mixture are shown in Table 27. The kneaded mixture was subjected to the "sterility test" in accordance with the general test procedure of the Japanese Pharmacopoeia. As a result, it was confirmed that the kneaded mixture was in the sterilized condition.

### Comparative Example 10

For comparison, the same powder and liquid as those used in Test Example 10 were charged at a powder-liquid ratio of 2.0 in a commercially available mortar for medical use and then manually kneaded for one minute, using a pestle. For comparison, the results of the hardening time and the compression strength are shown in Table 27.

**TABLE 27**

| | Comparative Example 10 | Test Example 10 | | |
|---|---|---|---|---|
| Powder-liquid ratio | 2.0 | 2.0 | 2.5 | 3.0 |
| Hardening time (min.) | 7.5 | 7.0 | 6.0 | 5.5 |
| Compression strength (MPa) | 53.0* | 62.2* | 65.9 | 73.0 |

| | | | | |
|---|---|---|---|---|
| *: There is a statistical significant difference in the results of the compression strength at a powder-liquid ratio of 2.0 of Comparative Example 10 and Test Example 10 (t-test, significance level: 5%). | | | | |

The compression strength and the hardening time were measured in accordance with JIS T6602 "Dental Zinc Phosphate Cement". The compression strength was measured in the wet condition after dipping a sample (7 mm φ × 14 mmL) in a simulated body fluid with the composition shown in Table 3 for 7 days. The loading speed was set to 0.5 mm/min. and a testing machine, Model 4455, manufactured by INSTRON CO.

As is apparent from the results shown in Table 26 and Table 27, the biomedical kneaded cement mixtures of Test Examples 9 and 10 prepared by the process of the present invention can be prepared within a shorter time as compared with the kneaded mixtures of Comparative Examples 9 and 10 prepared by a manual process (using a mortar) and the powder can be kneaded with the liquid at a high powder-liquid ratio and the hardening time of the resulting biomedical kneaded cement mixture is reduced as compared with Comparative Examples 9 and 10. In Test Example 9, biomedical kneaded cement mixtures capable of being discharged through a needle could be prepared at the powder-liquid ratio within a range from 1.5 to 3.0. As is apparent from the results of Table 27, the biomedical kneaded cement mixture of Test Example 10 prepared by the process of the present invention achieves a higher compression strength on hardening as compared with the kneaded mixture of Comparative Example 10 prepared by a manual process. As described in Test Example 10, a biomedical kneaded cement mixture could be prepared in the sterilized condition using a double vessel.

In Test Examples 9 and 10, various biomedical kneaded cement mixtures were prepared by varying the powder-liquid ratio and the kneading conditions (revolution speed and rotation speed) and the results of the description test, the discharge test, the hardening time and the compression strength were compared with those of the kneaded mixtures of Comparative Examples 9 and 10 prepared by a manual process (mortar). The results are summarized in Table 28 to Table 31.

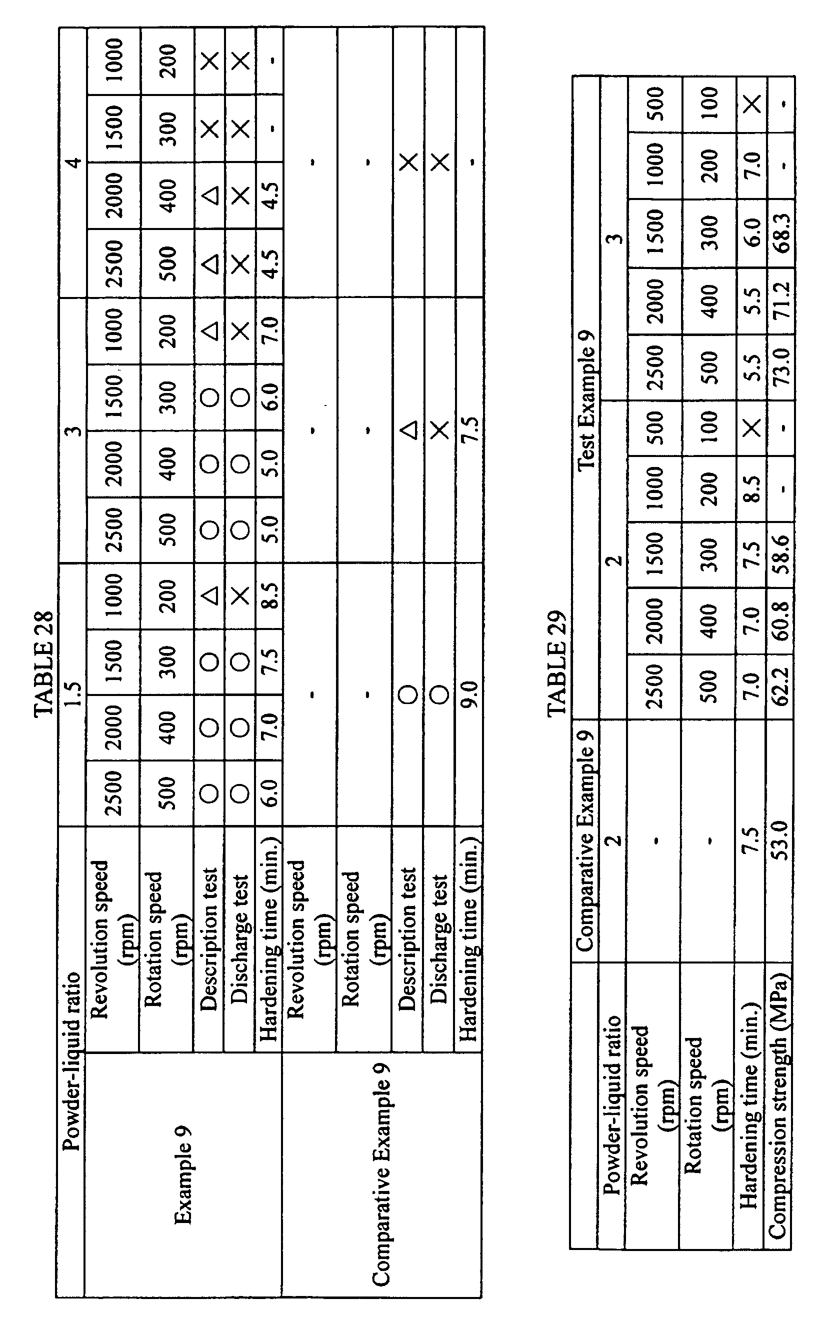

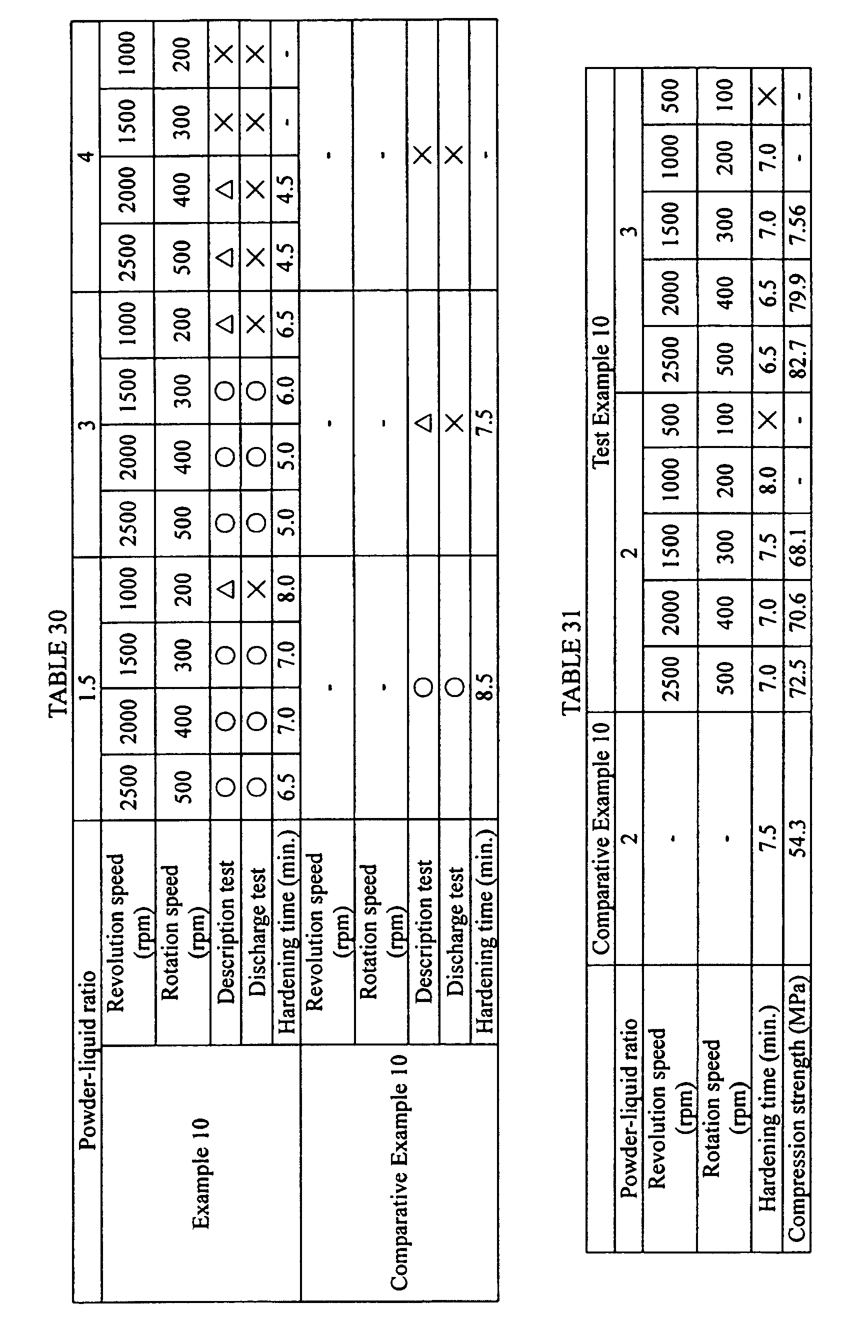

### [Test with respect to a powder composition which contains a pyrophosphate and has a specific surface area within a range from 0.3 to 2.0 m²/g]

### Test Example 11

95% of α-type tricalcium phosphate synthesized in Synthesis Example 3 was mixed with 5% of commercially available dicalcium phosphate (manufactured by Wako Pure Chemicals Industries, Ltd., reagent grade) to obtain a calcium phosphate powder. To 100 parts by weight of this calcium phosphate powder, 0.2 parts by weight of sodium pyrophosphate (manufactured by JUNSEI CHEMICAL Co., Ltd.) was added, followed by mixing using a grinder to obtain a powder composition. The specific surface area of the resulting powder composition was measured by MONOSORB, Model MS-13, manufactured by Yuasa Ionics Co., Ltd. As a result, it was 0.82 m²/g.

An aqueous solution, as a hardening liquid, was prepared by dissolving 20% by weight of disodium succinate hexahydrate (manufactured by JUNSEI CHEMICAL Co., Ltd.) and 5% by weight of sodium chondroitin sulfate (manufactured by SEIKAGAKU CORPORATION, injection grade ND product).

10 g of the powder composition was mixed with the liquid at a powder-liquid ratio (P/L) of 3.0, 3.5, 4.0, 4.5, 5.0 or 5.5, and then the mixture was charged in a vessel for exclusive use of "AWATORI RENTARO" (trade name, manufactured by THINKY Corp., specification: revolution speed of 2000 rpm and rotation speed of 400 rpm) and was kneaded for 15 seconds. The results of the description test, the discharge test, and the hardening time of the resulting biomedical kneaded cement mixture are shown in Table 32.

### Comparative Example 11

For comparison, the same powder and liquid as those used in Test Example 11 were charged in a commercially available mortar for medical use and then manually kneaded using a pestle. One minute was required to obtain a uniform paste. For comparison with Test Example 11, the results of the description test, the discharge test and the hardening time are shown in Table 32.

**TABLE 32**

| Powder-liquid ratio | | 3.0 | 3.5 | 4.0 | 4.5 | 5.0 | 5.5 |
|---|---|---|---|---|---|---|---|
| Test Example 11 | Description test | ○ | ○ | ○ | ○ | Δ | × |
| | Discharge test | ○ | ○ | ○ | ○ | × | × |
| | Hardening time | 6.5 min. | 6.0 min. | 5.0 min. | 4.5 min. | 4.5 min. | - |
| Comparative Example 11 | Description test | ○ | Δ | × | × | × | × |
| | Discharge test | Δ | × | × | × | × | × |
| | Hardening time | 8.5 min. | 7.0 min. | - | - | - | - |

### Test Example 12

### (Preparation of powder)

75.0% by weight of α-type tricalcium phosphate synthesized in Synthesis Example 1, 20.0% by weight of tetracalcium phosphate of Synthesis Example 2 and 5.0% by weight of commercially available dicalcium phosphate (manufactured by Wako Pure Chemicals Industries, Ltd.) were mixed to obtain a calcium phosphate powder. To 100 parts by weight of the calcium phosphate powder, 0.2 parts by weight of sodium pyrophosphate (manufactured by JUNSEI CHEMICAL Co., Ltd.) was added, followed by mixing to yield a powder composition. The specific surface area of the powder composition was measured in the same manner as in Test Example 9. As a result, it was 1.27 m²/g.

### (Sterilization)

The powder composition was irradiated with γ-rays at a dose of 25 kGy and the liquid of Test Example 11 was also sterilized by filtration through a bacterial filter having a pore diameter of 0.22 µm (manufactured by NIHON PALL LTD.). A double vessel having a shape shown in Fig. 1 was prepared and an outer vessel (polycarbonate vessel, manufactured by Tochigi Seiko Co., Ltd.) was subjected to high pressure steam sterilization under the conditions of a temperature of 121 °C for 20 minutes, while an inner vessel (polypropylene vessel, manufactured by Tochigi Seiko Co., Ltd.) was sterilized in an ethylene oxide gas sterilizer (manufactured by Nitto Science Co., Ltd., under the trade name of "Model CL-30-B").

### (Kneading)

The inner vessel was filled with 10 g of the powder and the liquid at P/L of 3.0, 3.5 or 4.0 and the inner vessel was set in the outer vessel. Then, the outer vessel was set in a "SPINKLE" (trade name, manufactured by J. MORITA TOKYO MFG. CORP., specification: revolution speed of 2500 rpm and rotation speed of 500 rpm) and kneaded for 10 seconds.

### (Test)

The results of the hardening time and the strength test of the resulting biomedical kneaded cement mixture are shown in Table 33. The kneaded mixture was subjected to the "sterility test" in accordance with the general test procedure of the Japanese Pharmacopoeia. As a result, it has been confirmed that the kneaded mixture is in the sterilized condition.

### Comparative Example 12

For comparison, the same powder and liquid as those used in Test Example 12 were charged at a powder-liquid ratio of 2.0 in a commercially available mortar for medical use and then manually kneaded for one minute, using a pestle. For comparison, the results of the hardening time and the compression strength are shown in Table 33.

**TABLE 33**

| | Comparative Example 12 | Test Example 12 | | |
|---|---|---|---|---|
| Powder-liquid ratio | 3.0 | 3.0 | 3.5 | 4.0 |
| Hardening time (min.) | 7.0 | 6.0 | 5.5 | 5.0 |
| Compression strength (MPa) | 71.4* | 80.9* | 90.1 | 99.2 |

| | | | | |
|---|---|---|---|---|
| *: There is a statistical significant difference in the results of the compression strength at a powder-liquid ratio of 3.0 of Comparative Example 12 and Test Example 12 (t-test, significance level: 5%). | | | | |

As is apparent from the results shown in Table 32 and Table 33, the biomedical kneaded cement mixtures of Test Examples 11 and 12 prepared by the process of the present invention can be prepared within a shorter time as compared with the kneaded mixtures of Comparative Examples 11 and 12 prepared by a manual process (using a mortar) and the powder can be kneaded with the liquid at a high powder-liquid ratio and the hardening time of the resulting biomedical kneaded cement mixture can be reduced as compared with Comparative Examples 11 and 12. In Test Example 11, biomedical kneaded cement mixtures capable of being discharged through a needle could be prepared at the powder-liquid ratio within a range from 3.0 to 4.5. As is apparent from the results of Table 33, the biomedical kneaded cement mixture of Test Example 12 prepared by the process of the present invention achieves a higher compression strength on hardening as compared with the kneaded mixture of Comparative Example 12 prepared by a manual process. As described in Test Example 12, a biomedical kneaded cement mixture could be prepared in the sterilized condition using a double vessel.

In Test Examples 11 and 12, various kneaded cement mixtures were prepared by varying the powder-liquid ratio and the kneading conditions (revolution speed and rotation speed) and the results of the description test, the discharge test, the hardening time and the compression strength were compared with those of the kneaded mixtures of Comparative Examples 11 and 12 prepared by a manual process (mortar). The results are summarized in Table 34 to Table 37.

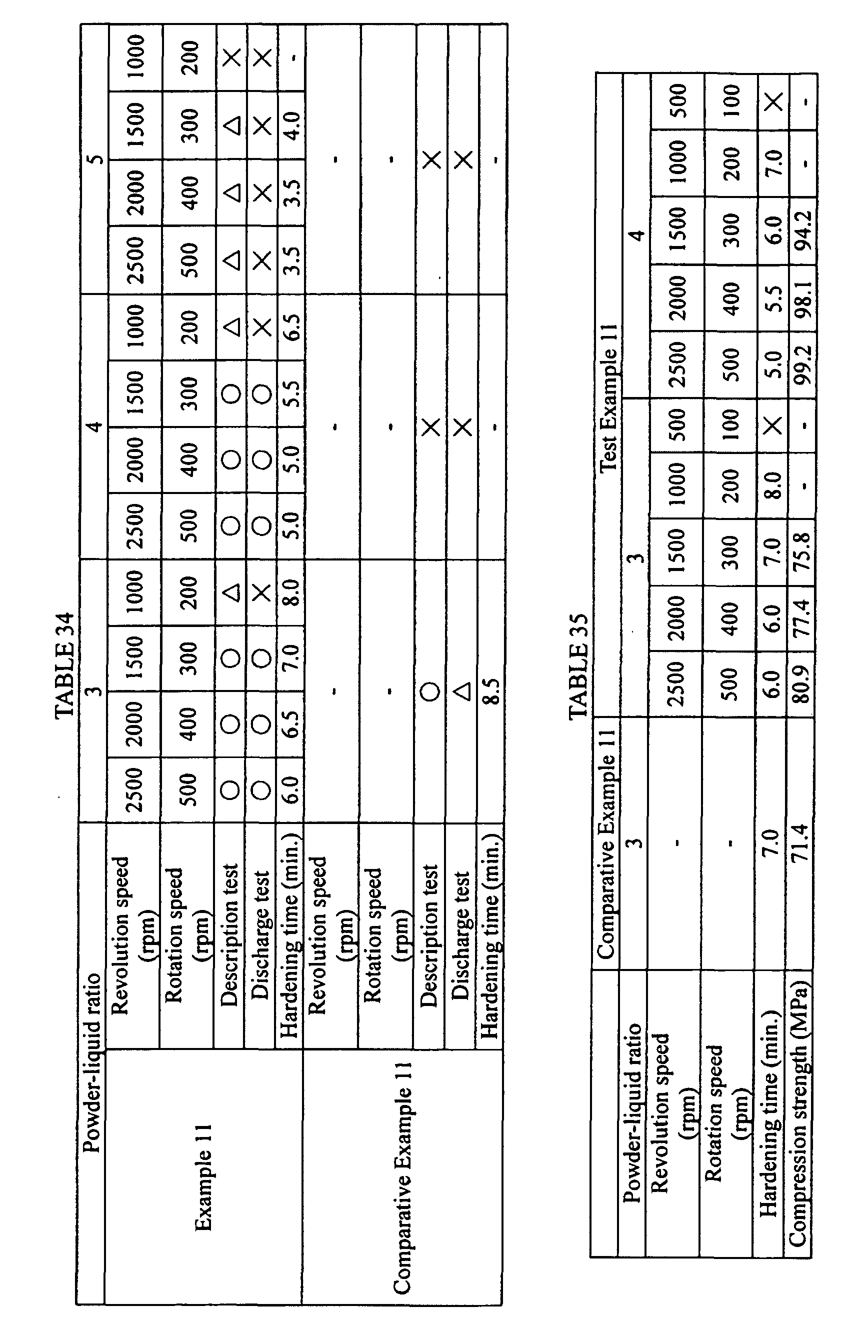

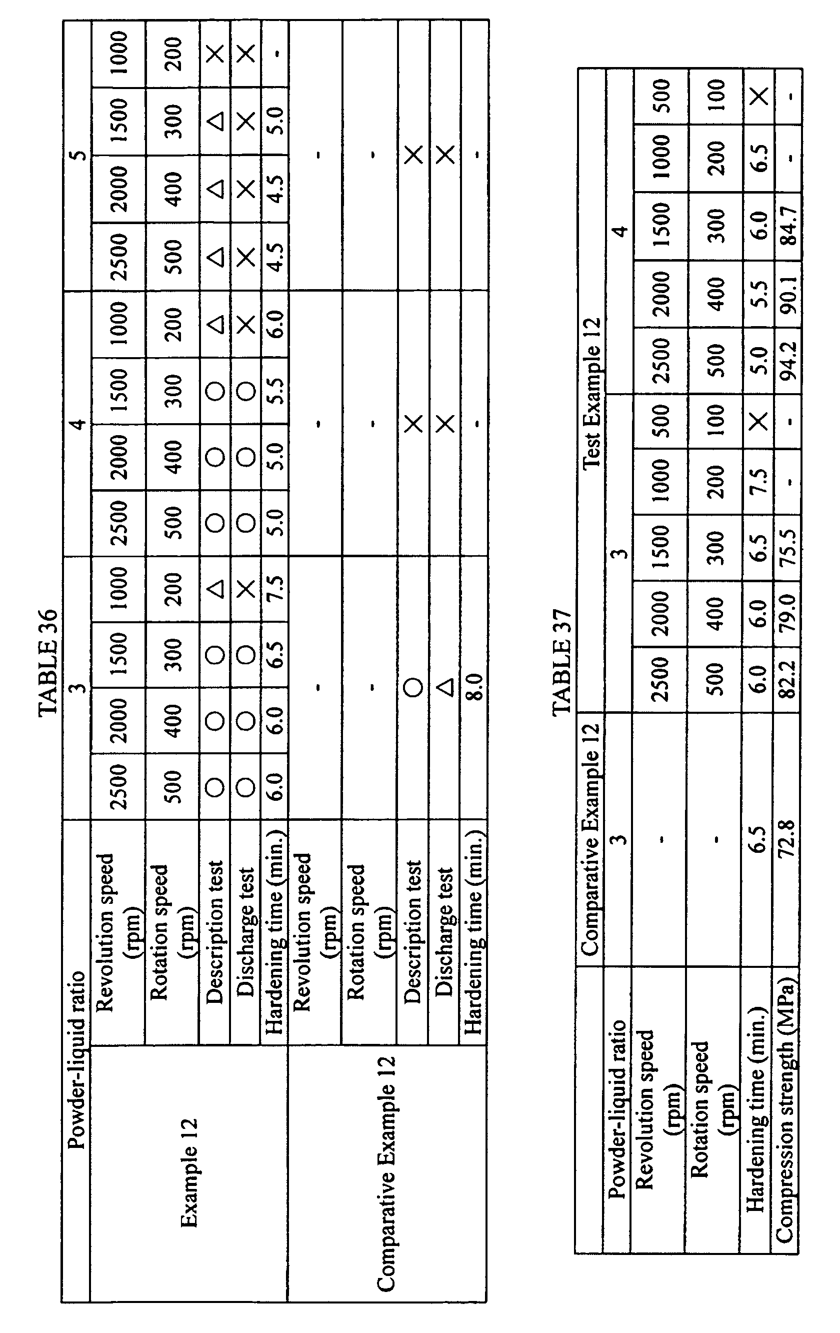

### INDUSTRIAL APPLICABILITY

According to the process of the present invention, by charging a powder composition and a hardening liquid in a vessel and kneading them by simultaneously revolving and rotating the vessel, it is made possible to uniformly knead the powder with the liquid at a high powder-liquid ratio at which uniform kneading could never have been conducted by a conventional manual kneading process, and to prepare a uniform kneaded cement mixture having a high viscosity at a high powder-liquid ratio in a simple manner at a low cost within a short time.

According to the present invention, since the kneaded cement mixture can be prepared without causing contamination with saprophytic bacteria and impurities, a kneaded mixture in the sterilized condition can be securely provided in a simple manner.

According to the present invention, even if a kneaded mixture is prepared at the same powder-liquid ratio, the strength on hardening is higher and the hardening time is shorter as compared with the kneaded mixture prepared by a conventional manual kneading process. Also according to the present invention, it is made possible to prepare a kneaded cement mixture having a high viscosity, which is useful as a biomedical prosthetic appliance that had been difficult to prepare using conventional processes, and to improve the utility of the biomedical cement.

## Claims

1. A process for preparing a kneaded calcium phosphate cement mixture, the process comprising the step of kneading a powder with a liquid by simultaneously revolving and rotating a vessel containing a material to be kneaded, which is prepared by mixing a powder composition containing a calcium phosphate powder with a hardening liquid.

2. A process for preparing a kneaded calcium phosphate cement mixture according to claim 1, wherein the powder is kneaded with the liquid by simultaneously revolving and rotating the vessel in the state of being inclined by 20 to 50 degrees.

3. A process for preparing a kneaded calcium phosphate cement mixture according to claim 1, wherein the powder is kneaded with the liquid by revolving the vessel at 1500 rpm or more while rotating it at 300 rpm in the state of being inclined by 20 to 50 degrees.

4. A process for preparing a kneaded calcium phosphate cement mixture according to claim 1, wherein the calcium phosphate powder is a powder of α-type tricalcium phosphate or tetracalcium phosphate, or a powder composition which contains at least one of α-type tricalcium phosphate and tetracalcium phosphate as an essential component and also contains dicalcium phosphate and monocalcium phosphate as an arbitrary component.

5. A process for preparing a kneaded calcium phosphate cement mixture according to claim 1, wherein the hardening liquid is an aqueous solution containing at least one selected from succinate, chondroitin sulfate, and hydrogensulfite.

6. A process for preparing a kneaded calcium phosphate cement mixture according to claim 1, wherein the vessel is a double vessel comprising an outer vessel and an inner vessel housed in the outer vessel.

7. A process for preparing a kneaded calcium phosphate cement mixture according to claim 6, wherein the outer vessel and the inner vessel are provided with a locking mechanism for preventing a relative position of the inner vessel housed in the outer vessel from changing.

8. A process for preparing a kneaded calcium phosphate cement mixture according to claim 7, wherein the inner vessel comprises a vessel body having a first opening for discharging a kneaded mixture and a second opening into which an extruding member for extruding the kneaded mixture in the inner vessel through the first opening, and a cover member which detachably seals the first opening and the second opening of the vessel body.

9. A process for preparing a kneaded calcium phosphate cement mixture according to claim 6, wherein kneading is conducted in the state where the outer vessel, the inner vessel and the material to be kneaded, which is contained in the inner vessel, are sterilized.

10. A kneaded calcium phosphate cement mixture obtained by mixing a calcium phosphate powder having a specific surface area of 0.3 to 2.0 m²/g with a hardening liquid in a weight mixing ratio of the powder to the liquid (P/L) of 3.0 to 5.0 and treating the mixture using the process of claim 1.

11. A kneaded calcium phosphate cement mixture obtained by mixing a calcium phosphate powder having a specific surface area of 2.0 to 4.0 m²/g with a hardening liquid in a weight mixing ratio of the powder to the liquid (P/L) of 1.5 to 3.5 and treating the mixture using the process of claim 1.

12. A kneaded calcium phosphate cement mixture according to claim 10 or 11, wherein a consistency thereof is from 18 to 22 mm.

13. A kneaded calcium phosphate cement mixture according to claim 10 or 11, wherein the amount of bubbles is 10% or less based on the volume of the kneaded mixture.

14. A hardened biomedical calcium phosphate bone filling cement material having a compression strength of 60 MPa or more, which is obtained by solidifying the kneaded calcium phosphate cement mixture of claim 10 or any one of claims 11 to 13.

15. A process for preparing a kneaded calcium phosphate cement mixture according to claim 1, wherein the powder composition is substantially composed only of a calcium phosphate powder.

16. A process for preparing a kneaded calcium phosphate cement mixture according to claim 1, wherein the powder composition contains a calcium phosphate powder and a magnesium-containing compound.

17. A process for preparing a kneaded calcium phosphate cement mixture according to claim 16, wherein the magnesium-containing compound is at least one of magnesium phosphate and magnesium sulfate.

18. A process for preparing a kneaded calcium phosphate cement mixture according to claim 1, wherein the powder composition contains a calcium phosphate powder and a pyrophosphate.

19. A process for preparing a kneaded calcium phosphate cement mixture according to claim 18, wherein the pyrophosphate is at least one of sodium pyrophosphate and potassium pyrophosphate.

20. A process for preparing a kneaded calcium phosphate cement mixture according to claim 18, wherein the powder composition further contains at least one of magnesium phosphate and magnesium sulfate.
